# EUROPEAN PATENT APPLICATION

(11) **EP 4 322 170 A1**
(43) Date of publication of application: **14.02.2024**
(21) Application number: 23184846.6
(22) Date of filing: 11.07.2023
(51) Int. Cl.: G16H 10/60, G16H 40/20, G16H 50/70, G06N 20/00

(54) **APPARATUSES, COMPUTER-IMPLEMENTED METHODS, AND COMPUTER PROGRAM PRODUCTS FOR IMPROVED HEALTH MONITOR DATA MONITORING**

(30) Priority: 10.08.2022 US 202217818872
(71) Applicant: Honeywell International Inc., Charlotte, NC 28202 (US)
(72) Inventor: SRINIVASAN, Ranganathan, Charlotte, 28202 (US); YADAV KATARU, Praneesh Kumar, Charlotte, 28202 (US)
(74) Representative: Haseltine Lake Kempner LLP

(57) **Abstract**

Embodiments of the present disclosure provide for improved health patches, monitoring systems, and implementations for improved capture and management of real-time health data. Embodiments facilitate receiving of real-time health data and maintaining the real-time health data in a particular manner based at least in part on one or more exception rules. Some embodiments receive real-time health data captured via a health monitor and store it to a local data store, apply the real-time health data to at least one exception rule that determines an exception indicator, and determine whether to transfer at least a portion of stored health data to an external system based at least in part on the exception indicator, for example for persistent, long-term storage and/or analysis.

## Description

### TECHNOLOGICAL FIELD

Embodiments of the present disclosure generally relate health monitors, and specifically to improved health monitors and associated system configurations for gathering, efficiently storing, and efficiently processing health data by such health monitors.

### BACKGROUND

Health monitors may be utilized to monitor health statistics of a particular user. A particular health monitor may capture various data, and sample at a rate such that the amount of captured data grows rapidly. In circumstances where multiple users are monitored, the amount of captured data may be significant, requiring large amounts of data storage systems to maintain and/or process. Applicant has discovered problems with current implementations of health monitors and associated configurations for gathering, efficiently storing, and efficiently processing health data. Through applied effort, ingenuity, and innovation, Applicant has solved many of these identified problems by developing embodied in the present disclosure, which are described in detail below.

### BRIEF SUMMARY

In general, embodiments of the present disclosure provide for improved health monitors and associated configurations for gathering, efficiently storing, and efficiently processing health data. Other implementations for improved health monitors and associated configurations for gathering, efficiently storing, and efficiently processing health data will be, or will become, apparent to one with skill in the art upon examination of the following figures and detailed description. It is intended that all such additional implementations be included within this description be within the scope of the disclosure, and be protected by the following claims.

In accordance with one aspect of the disclosure, a computer-implemented method for smart health monitor operation is provided. The example computer-implemented method may be executed via any of the computing device(s) embodied in hardware, software, firmware, and/or a combination thereof described herein. One example embodiment computer-implemented method includes receiving real-time health data captured via a health monitor. The example computer-implemented method further includes applying the real-time health data to at least one exception rule that determines an exception indicator. The example computer-implemented method further includes determining, based at least in part on the exception indicator, whether to transfer at least a portion of stored health data to an external system.

In some embodiments of the example computer-implemented method, wherein the computer-implemented method is executed on the health monitor.

In some embodiments of the example computer-implemented method, determining whether to transfer at least the portion of stored health data includes determining that the exception indicator indications an exception, identifying a complete stored health data set from a local datastore, and transferring the complete stored health data set from the local datastore.

In some embodiments of the example computer-implemented method, the portion of stored health data includes the real-time health data.

In some embodiments of the example computer-implemented method, the example computer-implemented method further including storing the real-time health data to a local datastore, wherein the apparatus is caused to continuously overwrite a defined historical timeseries of health data in the local datastore.

In some embodiments of the example computer-implemented method, determining whether to transfer at least the portion of stored health data includes determining that the exception indicator indicates an exception, in response to determining the exception indicator indicates the exception, transferring at least the portion of stored health data to the external system, and in response to determining the exception indicator indicates the exception, continuously transferring, in real-time, of each subsequently-received real-time health data. In some such embodiments of the example computer-implemented method, the example computer-implemented method further includes receiving a termination request, and terminating the continuous transfer in real-time of each subsequently-received real-time health data.

In some embodiments of the example computer-implemented method, the example computer-implemented method further including receiving annotated health data associated with the health monitor, training a health rules generation model based at least in part on the annotated health data, and generating the at least one exception rule utilizing the health rules generation model.

In some embodiments of the example computer-implemented method, the example computer-implemented method further including generating the at least one exception rule utilizing a health rules generation model, the health rules generation model including a specially trained artificial intelligence or a specially configured machine-learning model.

In some embodiments of the example computer-implemented method, where the real-time health data includes a plurality of data values associated with a plurality of different health parameters, the real-time health data including a particular timestamp.

In some embodiments of the example computer-implemented method, the example computer-implemented method further including causing the external data system to process at least the portion of stored health data to generate a health determination based at least in part on the portion of stored health data.

In some embodiments of the example computer-implemented method, determining whether to transfer at least the portion of stored health data include determining the exception indicator indicates no exception, and storing the real-time health data to a local datastore without transfer to the external system.

In some embodiments of the example computer-implemented method, the example computer-implemented method further including tracking a timestamp interval associated with a time since last data transfer or a time since last default data transfer, determining the timestamp interval satisfies a time interval threshold, in response to determining the timestamp interval satisfies the time interval threshold identifying at least default health data and transferring at least the default transfer data to the external data system.

In accordance with another aspect of the disclosure, an apparatus for smart health monitor operation is provided. In some embodiments the example apparatus includes at least one processor and at least one memory having computer-coded instructions stored thereon, where the computer-coded instructions cause the apparatus to perform each step of any one of the example computer-implemented methods described herein. In some embodiments another example apparatus includes means for performing each step of any one of the example computer-implemented methods described herein.

In accordance with another aspect of the disclosure, a computer program product for smart health monitor operation is provided. In some embodiments the example computer program product includes at least one non-transitory computer-readable storage medium having computer program code stored thereon that, in execution with at least one processor, configures the computer program product for performing each step of any one of the example computer-implemented methods described herein.

### BRIEF DESCRIPTION OF THE DRAWINGS

Having thus described the embodiments of the disclosure in general terms, reference now will be made to the accompanying drawings, which are not necessarily drawn to scale, and wherein:
FIG. 1 illustrates a block diagram of a system that may be specially configured within which embodiments of the present disclosure may operate;
FIG. 2 illustrates a block diagram of an example apparatus that may be specially configured in accordance with an example embodiment of the present disclosure;
FIG. 3 illustrates an example sequence diagram between computing devices for transferring health data based at least in part on an indicated exception in accordance with at least one example embodiment of the present disclosure;
FIG. 4 illustrates an example sequence diagram between computing devices for transferring health data based at least in part on a time threshold in accordance with at least one example embodiment of the present disclosure;
FIG. 5 illustrates an example data flow diagram for generation of exception rules in accordance with at least one example embodiment of the present disclosure;
FIG. 6 illustrates an example data flow diagram for determination of an exception indicator in accordance with at least one example embodiment of the present disclosure;
FIG. 7 illustrates an example data architecture for health data in accordance with at least one example embodiment of the present disclosure;
FIG. 8 illustrates a flowchart including example operations of an example computer-implemented process for managing storage of health data in accordance with at least one example embodiment of the present disclosure;
FIG. 9 illustrates a flowchart including example operations of an example computer-implemented process for determining whether to transfer health data based at least in part on an exception indicator in accordance with at least one example embodiment of the present disclosure;
FIG. 10 illustrates a flowchart including example operations of an example computer-implemented method for determining whether to transfer health data based at least in part on a time interval threshold in accordance with at least one example embodiment of the present disclosure;
FIG. 11 illustrates a flowchart including example operations of an example computer-implemented method for generating at least one exception rule in accordance with at least one example embodiment of the present disclosure; and
FIG. 12 illustrates a flowchart including example operations of an example computer-implemented method for continuously transferring real-time health data in accordance with at least one example embodiment of the present disclosure.

### DETAILED DESCRIPTION

Embodiments of the present disclosure now will be described more fully hereinafter with reference to the accompanying drawings, in which some, but not all, embodiments of the disclosure are shown. Indeed, embodiments of the disclosure may be embodied in many different forms and should not be construed as limited to the embodiments set forth herein, rather, these embodiments are provided so that this disclosure will satisfy applicable legal requirements. Like numbers refer to like elements throughout.

### Overview

In various contexts, data is monitored using remote health monitors. In one example context, one or more health monitor(s) are positioned on or otherwise associated with a particular patient. The health monitor(s) may collect data for any of a myriad health-related metrics. In some contexts, this data is collected and pushed for storage and/or analysis, for example by the user themselves and/or by one or more doctor(s) or other central authority. For example, in some contexts, data values for such health-related metrics are pushed to a central database that stores the values and enables a doctor to subsequently retrieve the data values and determine whether such values indicate a problem with the patient's health.

Such data collection and processing is vulnerable to multiple technical problems and deficiencies. Generally, data collection utilizing even one health monitor can generate a huge amount of data for diagnostic purposes. As such data is collected and stored, the amount of required storage/memory space required to store such data rapidly increases. Such data further increases rapidly, and can grow exponentially, as multiple health monitors are utilized and/or multiple health-related metrics are tracked simultaneously. For example, collection of ECG, SP02, temperature, and other health-related data can rapidly grow to more than 5GB in a 5-day period for a single patient (depending on collection frequency). Reducing such storage space required for collection and storage is desirable, particularly in contexts where paid services are utilized to store large amounts of data (e.g., cloud storage systems) that are paid based on the amount of storage space utilized and/or reserved. However, reducing collection frequency alone may decrease the likelihood that real-time health updates are captured quickly, which easily could make a difference between a patient seeking and/or treatment in time for them to be helped without issue, or suffering debilitating health effects or even death.

Embodiments of the present disclosure provide improvements for such collection and remote monitoring of health data. Embodiments of the present disclosure determine when to initiate a transfer of data collected and/or stored locally for a particular health monitor. Specifically, embodiments of the present disclosure utilize particular data-driven triggers to initiate data transfer of health data for persistent storage and/or subsequent processing. Such data-driven triggers include determination(s) based on exception rule(s) that, where satisfied, indicate particular portion(s) of health data represent abnormal, concerning, or other particular data value(s) for further analysis and/or storage. For example, captured health data that does not satisfy such exception rule(s) may indicate that such health data represents non-concerning or otherwise regular data value(s) not requiring further analysis and/or long-term storage.

As new health data is collected or otherwise captured, it may be stored locally in a manner that maintains a maximum size of locally stored data, for example where new health data overwrites previously captured data in a circumstance where maximum size of locally stored data is currently being stored locally, and/or a maximum length of time. Once triggered, only health data that is currently stored in a local storage may be transferred to a more permanent and scalable data storage, for example a cloud storage system. In this regard, such embodiments reduce or eliminate the amount of computing resources utilized to permanently maintain health data appropriate for such long-term storage and/or subsequent processing. By eliminating the amount of computing resources utilized to maintain health data that requires analysis, such embodiments may additionally reduce the number of computing system(s), computing device(s), and/or computing component(s) required to provide such computing resources, and in some embodiments additionally reduce costs associated with accessing, maintaining, and/or creating such computing system(s), computing device(s), and/or computing component(s). Using particular data-driven triggers (e.g., specially generated or determined exception rule(s)), some such embodiments provide these advantages with respect to computing resource usage without sacrificing accessibility and/or storage of health data pertinent to determination of a particular entity's health. In some embodiments, the maximum length of time or the maximum size associated with the local storage is determined by an external user, for example a user associated with an external system, a health practitioner, and/or the like.

One example context includes monitoring patients within a hospital setting, for example in a pre-operative or post-operative setting. In such contexts, monitoring of a patient's condition remains critical to ensure that the patient's health condition remains stable and/or otherwise not concerning. In some such circumstances, monitoring the patient is required to see if admission of the patient into a hospital, or if more critical care, is required based on the patient's health condition. In some such circumstances however, health data including particular monitored data value(s) may be captured at several samples per second, minute, and/or the like. Such data can quickly scale to significant amounts of data, requiring a significant amount of database space to store all such samples. Reducing the sampling is not desirable, since doing so reduces the likelihood that important changes in health is quickly detected, but similarly storing all such samples permanently in long-term storage even in circumstances in which such data is not changing much or indicating an exception to the user's health is similarly not desirable.

Embodiments of the present disclosure include smart health monitors, such as patches, and/or related supporting systems (e.g., linked user device(s)) that improve the monitoring and storing of health data. In some embodiments, an improved smart patch captures and processes real-time health data, as well as stores such data locally to the smart patch. The smart patch may subsequently process, or process via a linked support system, the real-time health data to determine whether such data indicates an exception to normal or expected data value(s) indicating a patient's health is normal. In a circumstance where an exception is indicated, then the real-time and/or stored health data may be transferred to an external datastore for long-term storage. If such data is collected and determined not to be an exception, however, such data may continue to be stored only locally, with limited amounts of data being pushed for long-term storing to reduce the total amount of data stored permanently via the long-term storage. Such embodiments enable long-term storage and/or remote analysis of reduced data by focusing on the data automatically determined as particularly pertinent, and without reducing the sampling rate utilized to consider whether real-time data indicates a concerning health circumstance for the patient. Thus, such embodiments reduce the amount of computing resources needed to sustainably monitor an entity, such as a patient, while simultaneously maintaining high frequency monitoring to ensure that the changes in an entity's condition can quickly and accurately be detected.

### Definitions

The term "health parameter" refers to electronically managed data representing any measurable metric representative of an aspect of the health of a particular entity.

The term "health data" refers to electronically managed data representing one or more data value(s) corresponding to one or more health parameter(s). Health data may include a single data value corresponding to a single health parameter, a plurality of data values corresponding to a single parameter, or a plurality of data values corresponding to a plurality of health parameters.

The term "real-time health data" refers to health data captured via a health monitor in real-time associated with a particular entity.

The term "stored health data" refers to one or more portions of health data stored in at least one datastore, where the health data is stored associated with a particular timestamp at which the health data was captured and/or otherwise stored.

The term "complete stored health data set" refers to all portions of health data store in at least one datastore.

The term "defined historical timeseries of health data" refers to one or more portions of stored health data arranged in chronological order based on each timestamp corresponding toa each portion of stored health data.

The term "subsequently-received real-time health data" refers to each portion of real-time health data captured subsequent to a particular trigger condition.

The term "health monitor" refers to any device embodied in hardware, software, firmware, and/or a combination thereof, that monitors at least one health parameter of a particular entity. Non-limiting examples of a health monitor include a blood pressure cuff, a glucometer, a pulse oximeter, a wearable tracker, one or more ECG probe(s), and a heart rate monitor.

The terms "support system" and "support device" with respect to a particular health monitor includes at least one computing device external to the particular health monitor that provides processing and/or storage functionality supporting operations of the health monitor. Non-limiting examples of a support system include a user smartphone communicatively coupled with a particular health monitor, a user laptop communicatively coupled with a particular health monitor, a monitoring system communicatively coupled with a particular health monitor, and a central monitoring system communicatively coupled with a particular health monitor, where any of such device(s) receives data value(s) captured by the health monitor for particular health parameter(s) and stores the data value(s) for the particular health parameter(s) in a local datastore on such device.

The term "exception" refers to a data-driven determination that data value(s) corresponding to one or more health parameter(s) indicate an abnormal health condition for a particular entity.

The term "exception rule" refers to electronically managed data defining a computer-implemented process for determining whether one or more data value(s) corresponding to one or more health parameter(s) indicate an exception for a particular entity. In some embodiments, different exception rules are applied to different entity. Non-limiting examples of an exception rule include an algorithm, a function, a truth table, a machine learning model, an artificial intelligence model, and/or a combination thereof.

The term "exception indicator" refers to electronically managed data indicating whether one or more portions of health data indicates an exception or indicates no exception.

The term "external system" refers to any one or more computing device(s) embodied in hardware, software, firmware, and/or a combination thereof, that is/are configured to store health data and is/are external to the health monitor.

The terms "local datastore" and "local data storage" refer to any one or more database(s) embodied in hardware, software, firmware, and/or any combination thereof, configured to store health data, and that are embodied within a health monitor and/or a companion device that is communicatively coupled with the health monitor to provide processing and/or storage functionality for supporting functionality of the health monitor.

The term "continuously" and "continuous" refers to an action that occurs at a particular rate. Any action that is "continuous" may be performed as quickly as possible, or at a defined rate, until a termination signal is received that terminates the continuous action.

The term "termination request" refers to electronically managed data indicating a signal for terminating continuous transfer of health data to an external system from the health monitor or a supporting device.

The term "annotated health data" refers to one or more portion(s) of health data including or linked with electronically managed data representing whether such one or more portion(s) of health data indicates an exception for a particular entity or all entities.

The term "health rules generation model" refers to one or more algorithmic, statistical, machine learning, and/or artificial intelligence model(s) that generate one or more exception rule(s) for one or more entity/entities.

The term "timestamp interval" refers to electronically managed data representing a length of time between a first time and a second time.

The term "time interval threshold" refers to a maximum length of time in which transfer of health data to an external system must occur before another transfer of health data is initiated.

The term "time since last data transfer" refers to a timestamp interval that represents a time since a transfer of health data to an external system was performed based at least in part on a determined or detected exception.

The term "time since last default data transfer" refers to a timestamp interval that represents a time since a transfer of health data to an external system was performed based at least in part on satisfaction of a time interval threshold.

### EXAMPLE SYSTEMS AND APPARATUSES OF THE DISCLOSURE

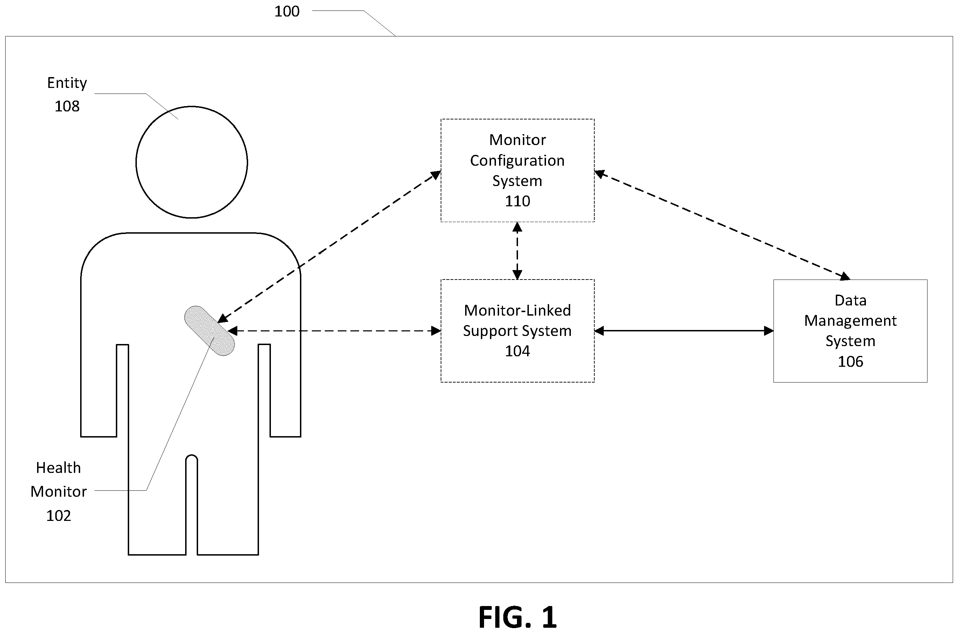

FIG. 1 illustrates a block diagram of a system that may be specially configured within which embodiments of the present disclosure may operate. Specifically, FIG. 1 illustrates a system 100 specially configured for improved smart health monitor operation, for example utilizing improved data collection and/or storage in accordance with at least one embodiment of the present disclosure. As depicted, the system 100 includes a health monitor 102, a data management system 106, an optional monitor-linked support system 104, and/or an optional monitor configuration system 110. The health monitor 102 is associated with, attached to, or otherwise coupled with an entity 108, such as a patient to which the health monitor 102 is attached via one or more diodes, sensors, and/or the like.

In some embodiments, the health monitor 102 includes one or more computing device(s) that captures, detects, reads, or otherwise receives real-time health data. The real-time health data may correspond to current data value(s) for health parameter(s) associated with the entity with which the health monitor 102 is attached, for example the entity 108. The health monitor 102 may be associated with capturing a single particular health parameter, or a plurality of health parameters simultaneously or in rapid succession. The health monitor 102 in some embodiments captures data value(s) for health parameter(s) at a defined sampling rate. In some embodiments, the health monitor 102 includes a plurality of sub-health monitors, each sub-health monitor embodying a different health monitor associated with capturing, reading, and/or otherwise receiving different data value(s) for different health parameter(s). In some embodiments, the health monitor 102 embodies a smart patch that monitors one or more particular health parameter(s).

The health monitor 102 is configured to enable storage of such collected real-time health data in a local storage. The local storage may be configured as a set size, such that a particular amount of data may be stored via the local storage in a continuous manner. In this regard, real-time health data may be stored to the local storage associated with a corresponding timestamp, forming a defined historical timeseries of health data as more samples of health data are captured. In some embodiments, the health monitor 102 pushes new real-time health data for storing via the local storage by overwriting one or more previously captured portions in the local storage, for example where the local storage overwrites the oldest data in the defined historical timeseries of health data.

In some embodiments, the local storage is embodied on the health monitor 102 itself. For example, in some embodiments, the health monitor 102 includes one or more memory/memories embodying at least a portion of the local storage. The health monitor 102 may write captured real-time health data, or otherwise received health data, to the memory/memories to store via the local storage. Alternatively or additionally, in some embodiments, the local storage is embodied in one or more external system(s), for example a monitor-linked support system 104 as described herein. In some embodiments, the health monitor 102 stores via the monitor-linked support system 104 by transmitting captured, collected, or otherwise received real-time health data to the external local storage for storing, for example via transmission to the monitor-linked support system.

The monitor configuration system 110 includes one or more computing device(s) embodied in hardware, software, firmware, and/or a combination thereof, that communicates with the health monitor 102 to configure one or more operational aspects of the health monitor 102. In some embodiments, the monitor configuration system 110 includes one or more application server(s), database server(s), and/or the like, external to the health monitor 102. In some embodiments, the health monitor 102 and the monitor configuration system 110 are communicatively coupled via one or more communications network(s). The communications network(s) may include wired communication network(s), wireless communication network(s), and/or a combination thereof.

In some embodiments, the monitor configuration system 110 is configured to transmit data utilized to configure one or more operational aspect(s) of the health monitor 102. In some embodiments, for example, the monitor configuration system 110 transmits data that configures a sampling rate of the health monitor 102. Alternatively or additionally, in some embodiments, the monitor configuration system 110 transmits data that configures the health monitor 102 to maintain and/or utilize particular exception rule(s). Alternatively or additionally, in some embodiments, the monitor configuration system 110 transmits data that configures the health monitor 102 to maintain and/or utilize one or more health rules generation model(s). Additionally or alternatively, in some embodiments, the monitor configuration system 110 transmits data that configures one or more threshold(s) utilized by the health monitor 102, for example a time interval threshold utilized to transmit stored health data and/or data derived therefrom at a default interval.

The monitor-linked support system 104 includes one or more computing device(s) embodied in hardware, software, firmware, and/or a combination thereof, that supports and/or facilitates the data processing, data collection, and/or data operation(s) of the health monitor 102. In some embodiments, the monitor-linked support system 104 includes or embodies a local datastore that stores real-time health data captured via the health monitor 102. In some embodiments, the monitor-linked support system 104 includes or is embodied by a user device linked to the health monitor 102. The monitor-linked support system 104 in some embodiments includes one or more specially configured software application(s) executing on one or more computing device(s), such as the user device, that provides such functionality for supporting the operations of the health monitor 102. For example, the monitor-linked support system 104 includes a software application executing on a user device that receives real-time health data transmitted from the health monitor 102 and stores such real-time health data in a local storage associated with a particular timestamp. Additionally or alternatively, in some embodiments the monitor-linked support system 104 provides access to previously stored health data, for example by enabling user access to data within the local storage and/or transmitted to a long-term storage. Non-limiting examples of the monitor-linked support system 104 include a smart phone, a tablet, a personal computer, a laptop, a central monitoring system, a server, and/or the like, that is communicatively coupled with the health monitor 102 over a short-range communications network, for example a wired connection, Bluetooth low energy, Zigbee, and/or the like, and configured via one or more specially configured software application(s) executing on said device(s). In some embodiments, the monitor-linked support system 104 includes or generates one or more user interface(s) that enables a user to perform such functionality, interact with the health monitor 102, and/or the like, for example via a companion app executing via the monitor-linked support system 104.

In some embodiments the health monitor 102 includes or embodies the monitor-linked support system 104. In some embodiments, the health monitor 102 includes a sub-system embodying the monitor-linked support system 104. In some such embodiments, the health monitor 102 communicates and/or functions without a monitor-linked support system 104. In this regard, in some such embodiments the monitor-linked support system 104 is entirely optional. Alternatively or additionally, in some embodiments, the monitor-linked support system 104 includes or is embodies the monitor configuration system 110.

The data management system 106 includes one or more computing device(s) embodied in hardware, software, firmware, and/or a combination thereof. In some embodiments, the data management system 106 includes one or more application server(s) that provide processing and/or operational functionality. Alternatively or additionally, in some embodiments the data management system 106 includes one or more database server(s) that provide long-term data storage, maintenance, and/or retrieval functionality. Non-limiting examples of a data management system 106 include at least one server, at least one personal computer, a health office or hospital centralized system, and/or the like.

In some embodiments, the data management system 106 stores received health data. For example, the health data may represent health data captured and/or transmitted via the health monitor 102, for example directly or indirectly via a monitor-linked support system 104. In some embodiments, the data management system 106 receives such health data and stores the health data in a long-term and/or persistent datastore, for example utilizing a cloud-based repository system.

In some embodiments, the data management system 106 is accessible via a particular provider, administrator, or other authority associated with the collected health data. For example, the data management system 106 in some embodiments is accessible to a health professional (e.g., via a corresponding client device utilized to connect to the data management system 106 during an authenticated session) to retrieve and/or view stored health data associated with a particular entity. It should be appreciated that the data management system 106 may be specially configured to enable data storage of a larger amount of data and/or for a longer time period than the health monitor 102 and/or associated monitor-linked support system 104. For example, the data management system 106 may permanently store received health data and/or store received health data for a certain, longer time interval (e.g., a period of years) than the health monitor 102 or associated monitor-linked support system.

In some embodiments, the data management system 106 is specially configured to perform one or more other data management function(s). For example, in some embodiments, the data management system 106 enables annotation of health data stored via the data management system 106, for example to indicate whether such health data represents a normal health condition of the entity or a concerning health condition of the entity. In some embodiments, the data management system 106 automatically or manually (e.g., in response to user input by a health professional for example) generates one or more exception rule(s) for use by a health monitor, such as the health monitor 102. In some embodiments, the data management system 106 executes one or more health rules generation model(s) that generate at least one exception rule associated with a particular entity (e.g., a patient) or a plurality of entities based on stored health data corresponding to such entities. In some embodiments, the data management system 106 determines exception rule(s), threshold(s), and/or other data to be utilized to configure the health monitor 102, and pushes such data directly to the health monitor 102 for use and/or indirectly through one or more other system(s), for example a monitor configuration system 110. In some embodiments, the data management system 106 includes or embodies the monitor configuration system 110.

In some embodiments, the data management system 106 is communicable over a long range communications network. For example, in some embodiments, the data management system 106 is communicable with the health monitor 102 and/or the monitor-linked support system 104 via Wi-Fi, the Internet, or another public or private communications network that may span a large area. In some such embodiments, the data management system 106 may be communicable over such a longer-range communications network as the health monitor 102 communicates with a monitor-linked support system 104 via a shorter-range communications network, for example Bluetooth low energy or the like. Alternatively or additionally, in some embodiments the health monitor 102 and/or the monitor-linked support system 104 any wired, wireless, long-range, and/or short-range wireless communications network(s). The data management system 106 may communicate with other system(s), for example the monitor configuration system 110, via any of such communications network(s).

The communications network(s) as described in some embodiments is embodied in any of a myriad of network configurations. In some embodiments, the communications network(s) embody a public network (e.g., the Internet). In some embodiments, the communications network(s) embodies a private network (e.g., an internal, localized, or closed-off network between particular devices). In some other embodiments, the communications network(s) embody a hybrid network (e.g., a network enabling internal communications between particular connected devices and external communications with other devices). The communications network(s) in some embodiments includes one or more base station(s), relay(s), router(s), switch(es), cell tower(s), communications cable(s) and/or associated routing station(s), and/or the like. In some embodiments, the communications network(s) includes one or more user controlled computing device(s) (e.g., a user owner router and/or modem) and/or one or more external utility devices (e.g., Internet service provider communication tower(s) and/or other device(s)).

The computing device(s) each may communicate over a whole or a portion of one or more communications network(s). For example, each of the components of the system communicatively coupled to transmit data to and/or receive data from, for example, one another over the same or different wireless or wired networks embodying the communications network(s). Such configuration(s) include, without limitation, a wired or wireless Personal Area Network (PAN), Local Area Network (LAN), Metropolitan Area Network (MAN), Wide Area Network (WAN), and/or the like. Additionally, while FIG. 1 illustrate certain system entities as separate, standalone entities communicating over the communications network(s), the various embodiments are not limited to this particular architecture. In other embodiments, one or more computing entities share one or more components, hardware, and/or the like, or otherwise are embodied by a single computing device such that connection(s) between the computing entities are over the communications network(s) are altered and/or rendered unnecessary. Alternatively or additionally still, in some embodiments the communications network(s) enable communication to one or more other computing device(s) not depicted, for example client device(s) for accessing functionality of any of the subsystems therein via native and/or web-based application(s), and/or the like.

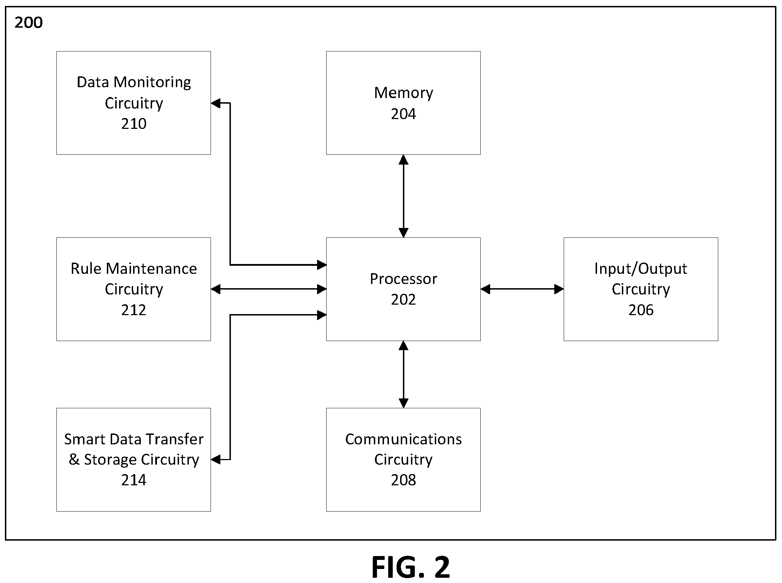

FIG. 2 illustrates a block diagram of an example apparatus that may be specially configured in accordance with an example embodiment of the present disclosure. Specifically, FIG. 2 illustrates an example improved monitoring apparatus 200 ("apparatus 200") specially configured in accordance with at least some example embodiments of the present disclosure. In some embodiments, the health monitor 102 and/or a portion thereof is embodied by one or more system(s), such as the apparatus 200 as depicted and described in FIG. 2. Alternatively or additionally, in some embodiments, a single computing system embodies a combination of the health monitor 102 in conjunction with a monitor-linked support system 104, such as the apparatus 200 as depicted and described in FIG. 2. The apparatus 200 includes processor 202, memory 204, input/output circuitry 206, communications circuitry 208, data monitoring circuitry 210, rule maintenance circuitry 212, and smart data transfer & storage circuitry 214. In some embodiments, the apparatus 200 is configured, using one or more of the sets of circuitry 202, 204, 206, 208, 210, 212, and/or 214, to execute and perform the operations described herein.

In general, the terms computing entity (or "entity" in reference other than to a user), device, system, and/or similar words used herein interchangeably may refer to, for example, one or more computers, computing entities, desktop computers, mobile phones, tablets, phablets, notebooks, laptops, distributed systems, items/devices, terminals, servers or server networks, blades, gateways, switches, processing devices, processing entities, set-top boxes, relays, routers, network access points, base stations, the like, and/or any combination of devices or entities adapted to perform the functions, operations, and/or processes described herein. Such functions, operations, and/or processes may include, for example, transmitting, receiving, operating on, processing, displaying, storing, determining, creating/generating, monitoring, evaluating, comparing, and/or similar terms used herein interchangeably. In one embodiment, these functions, operations, and/or processes can be performed on data, content, information, and/or similar terms used herein interchangeably. In this regard, the apparatus 200 embodies a particular, specially configured computing entity transformed to enable the specific operations described herein and provide the specific advantages associated therewith, as described herein.

Although components are described with respect to functional limitations, it should be understood that the particular implementations necessarily include the use of particular computing hardware. It should also be understood that in some embodiments certain of the components described herein include similar or common hardware. For example, in some embodiments two sets of circuitry both leverage use of the same processor(s), network interface(s), storage medium(s), and/or the like, to perform their associated functions, such that duplicate hardware is not required for each set of circuitry. The use of the term "circuitry" as used herein with respect to components of the apparatuses described herein should therefore be understood to include particular hardware configured to perform the functions associated with the particular circuitry as described herein.

Particularly, the term "circuitry" should be understood broadly to include hardware and, in some embodiments, software for configuring the hardware. For example, in some embodiments, "circuitry" includes processing circuitry, storage media, network interfaces, input/output devices, and/or the like. Alternatively or additionally, in some embodiments, other elements of the apparatus 200 provide or supplement the functionality of another particular set of circuitry. For example, the processor 202 in some embodiments provides processing functionality to any of the sets of circuitry, the memory 204 provides storage functionality to any of the sets of circuitry, the communications circuitry 208 provides network interface functionality to any of the sets of circuitry, and/or the like.

In some embodiments, the processor 202 (and/or co-processor or any other processing circuitry assisting or otherwise associated with the processor) is/are in communication with the memory 204 via a bus for passing information among components of the apparatus 200. In some embodiments, for example, the memory 204 is non-transitory and may include, for example, one or more volatile and/or non-volatile memories. In other words, for example, the memory 204 in some embodiments includes or embodies an electronic storage device (e.g., a computer readable storage medium). In some embodiments, the memory 204 is configured to store information, data, content, applications, instructions, or the like, for enabling the apparatus 200 to carry out various functions in accordance with example embodiments of the present disclosure.

The processor 202 may be embodied in a number of different ways. For example, in some example embodiments, the processor 202 includes one or more processing devices configured to perform independently. Additionally or alternatively, in some embodiments, the processor 202 includes one or more processor(s) configured in tandem via a bus to enable independent execution of instructions, pipelining, and/or multithreading. The use of the terms "processor" and "processing circuitry" should be understood to include a single core processor, a multi-core processor, multiple processors internal to the apparatus 200, and/or one or more remote or "cloud" processor(s) external to the apparatus 200.

In an example embodiment, the processor 202 is configured to execute instructions stored in the memory 204 or otherwise accessible to the processor. Alternatively or additionally, the processor 202 in some embodiments is configured to execute hard-coded functionality. As such, whether configured by hardware or software methods, or by a combination thereof, the processor 202 represents an entity (e.g., physically embodied in circuitry) capable of performing operations according to an embodiment of the present disclosure while configured accordingly. Alternatively or additionally, as another example in some example embodiments, when the processor 202 is embodied as an executor of software instructions, the instructions specifically configure the processor 202 to perform the algorithms embodied in the specific operations described herein when such instructions are executed.

As one particular example embodiment, the processor 202 is configured to perform various operations associated with monitoring and facilitating improved storage and transfer of health data. In some embodiments, the processor 202 includes hardware, software, firmware, and/or a combination thereof, that captures, collects, gathers, or otherwise monitors real-time health data. Additionally or alternatively, in some embodiments, the processor 202 includes hardware, software, firmware, and/or a combination thereof, that facilitates storage of captured real-time health data to a local storage. Additionally or alternatively, in some embodiments, the processor 202 includes hardware, software, firmware, and/or a combination thereof, that generates an exception indicator representing whether real-time health data satisfies one or more exception rule(s). Additionally or alternatively, in some embodiments, the processor 202 includes hardware, software, firmware, and/or a combination thereof, that facilitates transfer of real-time health data and/or at least a portion of stored health data to an external system based at least in part on an exception indicator representing satisfaction of one or more exception rule(s). Additionally or alternatively, in some embodiments, the processor 202 includes hardware, software, firmware, and/or a combination thereof, that facilitates continuous transfer of subsequently captured real-time health data to an external system until a termination signal is received. Additionally or alternatively, in some embodiments, the processor 202 includes hardware, software, firmware, and/or a combination thereof, that transfers real-time health data and/or at least a portion of stored health data in response to a determination that a particular time interval threshold is satisfied, for example based at least in part on a time since a last data transfer or a time since a last default data transfer. Additionally or alternatively, in some embodiments, the processor 202 includes hardware, software, firmware, and/or a combination thereof, that generates one or more exception rule(s) utilizing one or more health rule generation model(s).

In some embodiments, the apparatus 200 includes input/output circuitry 206 that provides output to the user and, in some embodiments, to receive an indication of a user input. In some embodiments, the input/output circuitry 206 is in communication with the processor 202 to provide such functionality. The input/output circuitry 206 may comprise one or more user interface(s) and in some embodiments includes a display that comprises the interface(s) rendered as a web user interface, an application user interface, a user device, a backend system, or the like. In some embodiments, the input/output circuitry 206 also includes a keyboard, a mouse, a joystick, a touch screen, touch areas, soft keys a microphone, a speaker, or other input/output mechanisms. The processor 202 and/or input/output circuitry 206 comprising the processor may be configured to control one or more functions of one or more user interface elements through computer program instructions (e.g., software and/or firmware) stored on a memory accessible to the processor (e.g., memory 204, and/or the like). In some embodiments, the input/output circuitry 206 includes or utilizes a user-facing application to provide input/output functionality to a client device and/or other display associated with a user.

In some embodiments, the apparatus 200 includes communications circuitry 208. The communications circuitry 208 includes any means such as a device or circuitry embodied in either hardware or a combination of hardware and software that is configured to receive and/or transmit data from/to a network and/or any other device, circuitry, or module in communication with the apparatus 200. In this regard, in some embodiments the communications circuitry 208 includes, for example, a network interface for enabling communications with a wired or wireless communications network. Additionally or alternatively in some embodiments, the communications circuitry 208 includes one or more network interface card(s), antenna(s), bus(es), switch(es), router(s), modem(s), and supporting hardware, firmware, and/or software, or any other device suitable for enabling communications via one or more communications network(s). Additionally or alternatively, the communications circuitry 208 includes circuitry for interacting with the antenna(s) and/or other hardware or software to cause transmission of signals via the antenna(s) or to handle receipt of signals received via the antenna(s). In some embodiments, the communications circuitry 208 enables transmission to and/or receipt of data from a client device, capture device, and/or other external computing device in communication with the apparatus 200.

In some embodiments, the apparatus 200 includes data monitoring circuitry 210. The data monitoring circuitry 210 includes hardware, software, firmware, and/or a combination thereof, that supports various functionality associated with monitoring real-time health data. For example, in some embodiments, the data monitoring circuitry 210 includes one or more probe(s), sensor(s), and/or specially configured device(s) that capture electrical signal(s) representing data value(s) for particular health parameter(s) at one or more sampling rate(s). Additionally or alternatively, in some embodiments, the data monitoring circuitry 210 includes hardware, software, firmware, and/or a combination thereof, that generates real-time health data interpreted from captured electrical signal(s), for example via one or more probe(s). Additionally or alternatively, in some embodiments, the data monitoring circuitry 210 includes hardware, software, firmware, and/or a combination thereof, that causes storage of captured real-time health data to a local storage associated with a particular corresponding timestamp. Additionally or alternatively, in some embodiments, the data monitoring circuitry 210 includes hardware, software, firmware, and/or a combination thereof, that overwrites existing data of a defined historical timeseries of health data. In some embodiments, data monitoring circuitry 210 includes a separate processor, specially configured field programmable gate array (FPGA), or a specially programmed application specific integrated circuit (ASIC).

In some embodiments, the apparatus 200 includes rule maintenance circuitry 212. The rule maintenance circuitry 212 includes hardware, software, firmware, and/or a combination thereof, that supports various functionality associated with generation and/or maintenance of exception rule(s). For example, in some embodiments, the rule maintenance circuitry 212 includes hardware, software, firmware, and/or a combination thereof that maintains at least one exception rule stored accessible to the apparatus 200. Alternatively or additionally, in some embodiments, the rule maintenance circuitry 212 includes hardware, software, firmware, and/or a combination thereof, that receives, stores, and/or maintains one or more health rules generation model(s). Alternatively or additionally, in some embodiments, the rule maintenance circuitry 212 includes hardware, software, firmware, and/or a combination thereof, that generates at least one exception rule, for example using a health rules generation model. Alternatively or additionally, in some embodiments, the rule maintenance circuitry 212 includes hardware, software, firmware, and/or a combination thereof, that applies data to one or more health rules generation model(s) to generate exception rule(s). Alternatively or additionally, in some embodiments, the rule maintenance circuitry 212 includes hardware, software, firmware, and/or a combination thereof, that communicates with an external system to receive, update, and/or otherwise exception rule(s) and/or health rules generation model(s). In some embodiments, rule maintenance circuitry 212 includes a separate processor, specially configured field programmable gate array (FPGA), or a specially programmed application specific integrated circuit (ASIC).

In some embodiments, the apparatus 200 includes smart data transfer & storage circuitry 214. For example, in some embodiments the smart data transfer & storage circuitry 214 includes hardware, software, firmware, and/or a combination thereof, that generates an exception indicator based at least in part on at least one exception rule, such as by applying health data to the at least one exception rule. Additionally or alternatively, in some embodiments, the smart data transfer & storage circuitry 214 includes hardware, software, firmware, and/or a combination thereof, that determine whether an exception indicator indicates an exception or no exception. Additionally or alternatively, in some embodiments, the smart data transfer & storage circuitry 214 includes hardware, software, firmware, and/or a combination thereof, that tracks a time interval since a last exception-triggered transfer, and/or a time interval since a last default transfer. Additionally or alternatively, in some embodiments, the smart data transfer & storage circuitry 214 includes hardware, software, firmware, and/or a combination thereof, that determines whether a particular timestamp interval satisfies a time interval threshold. Additionally or alternatively, in some embodiments, the smart data transfer & storage circuitry 214 includes hardware, software, firmware, and/or a combination thereof, that retrieves at least a portion of health data for transferring, for example a complete stored health data set from a local datastore, real-time health data received via the apparatus 200, subsequently-received real-time health data, a default health data portion, and/or the like. Additionally or alternatively, in some embodiments, the smart data transfer & storage circuitry 214 includes hardware, software, firmware, and/or a combination thereof, that transfers particular portion(s) of health data to an external system, for example transferring default health data, real-time health data, at portion of stored health data, and/or a complete stored health data set. Additionally or alternatively, in some embodiments, the smart data transfer & storage circuitry 214 includes hardware, software, firmware, and/or a combination thereof, that continuously transfers subsequently-received real-time health data to an external system. In some embodiments, the smart data transfer & storage circuitry 214 includes a separate processor, specially configured field programmable gate array (FPGA), or a specially programmed application specific integrated circuit (ASIC).

Additionally or alternatively, in some embodiments, two or more of the sets of circuitries 202-214 are combinable. Alternatively or additionally, in some embodiments, one or more of the sets of circuitry perform some or all of the functionality described associated with another component. For example, in some embodiments, two or more of the sets of circuitry 202-214 are combined into a single module embodied in hardware, software, firmware, and/or a combination thereof. Similarly, in some embodiments, one or more of the sets of circuitry, for example the data monitoring circuitry 210, rules maintenance circuitry 212, and/or smart data transfer & storage circuitry 214, is/are combined with the processor 202, such that the processor 202 performs one or more of the operations described above with respect to each of these sets of circuitry 210-214.

### EXAMPLE DATA FLOWS OF THE DISCLOSURE

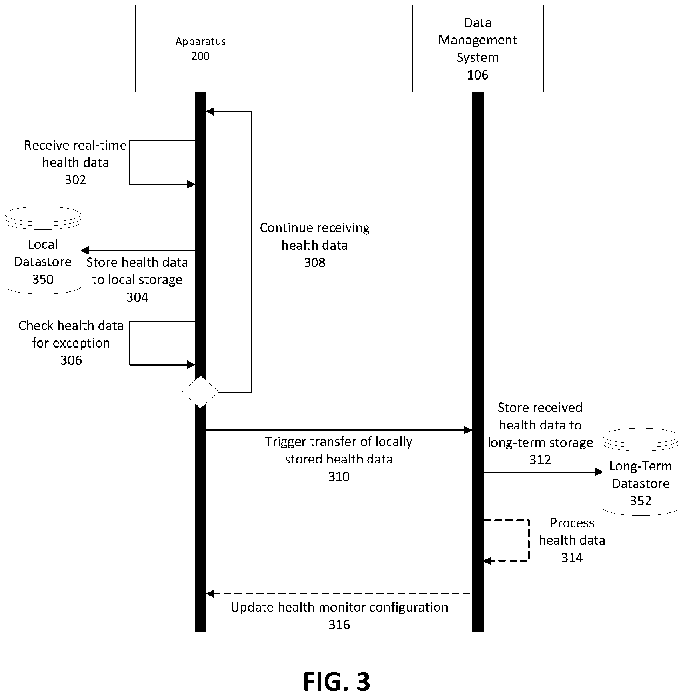

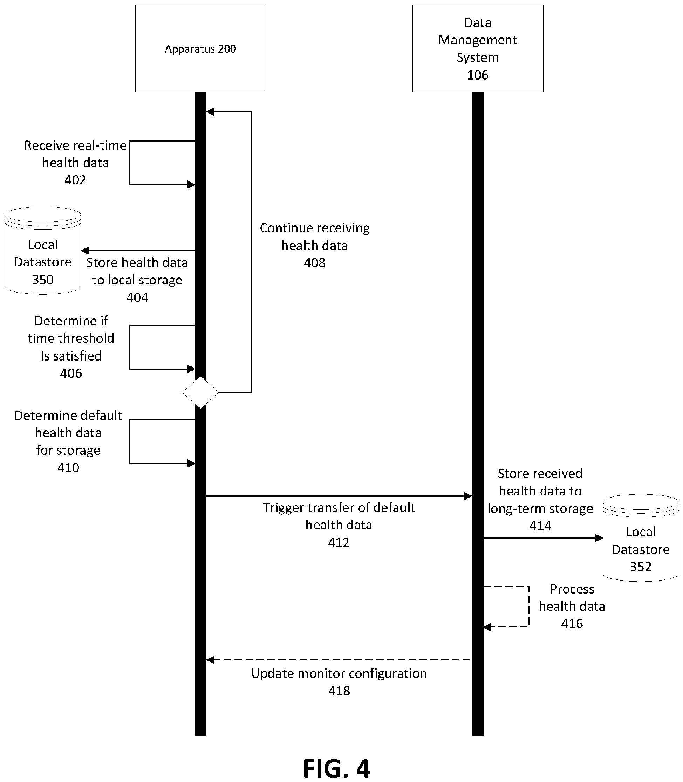

Having described example systems and apparatuses of the disclosure, example data flows between data systems of data objects of a particular architecture will now be discussed. In some embodiments, one or more specially configured computing device(s) is configured via hardware, software, firmware, and/or any combination thereof, to perform such operations for smart health monitor operation. For example, in some embodiments, the health monitor 102 and/or monitor-linked support system 104 embodied by the apparatus 200 interacts with one or more other system(s) and/or device(s) as part of the data flow(s) as depicted and described in FIGS. 3 and 4. It will be appreciated that each data flow may represent a computer-implemented process performed via one or more computing device(s), such as those depicted to perform the particular steps of the data flow.

FIG. 3 illustrates an example sequence diagram between computing devices for transferring health data based at least in part on an indicated exception in accordance with at least one example embodiment of the present disclosure. Specifically, FIG. 3 illustrates a data flow between apparatus 200 and an external data management system 106 for transfer of health data in a circumstance where an exception indicator represents an exception, and continuing collection where the exception indicator represents no exception.

As illustrated, at step 302 the apparatus 200 receives real-time health data. In some embodiments, the apparatus 200 captures the real-time health data. In some embodiments, the apparatus 200 receives the real-time health data as transmitted from a health monitor.

At step 304, the apparatus 200 stores the real-time health data to a local storage. As illustrated, the apparatus 200 stores the health data to local datastore 350. In some embodiments, the local datastore 350 is embodied as a subcomponent or subsystem of the apparatus 200. In some embodiments, the local datastore 350 is embodied as an external component or system local to and/or communicable with the apparatus 200. In some embodiments, the apparatus 200 only stores the data to the local datastore in a circumstance where an exception is not detected, for example at step 306 as described herein.

At step 306, the apparatus 200 checks the health data for an exception. In some embodiments, the apparatus 200 checks the real-time health data to determine whether the real-time health data indicates an exception. Alternatively or additionally, in some embodiments the apparatus 200 checks real-time health data and/or stored health data to determine whether such data indicates an exception.

In some embodiments, the apparatus 200 applies the real-time health data (and/or at least a portion of stored health data) to at least one exception rule. The exception rule determines an exception indicator, which represents whether such data indicates an exception or no exception. For example, in some embodiments, the exception rule(s) determine whether data value(s) of the real-time health data-alone or in combination with one or more portion(s) of stored health data-falls outside of a normally expected range for such data value(s) alone or in combination. Alternatively or additionally, in the context of a healthcare patient for example, the exception rule(s) may indicate whether the real-time health data, alone or in conjunction with at least a portion of stored health data, indicates a concerning health condition associated with the patient.

In a circumstance where an exception is not indicated, for example by the apparatus 200 determining an exception indicator represents no exception, the apparatus 200 at step 308 continues receiving real-time health data. In this regard, the apparatus 200 may return to step 302 to continue receiving new portion(s) of real-time health data. It will be appreciated that in this regard, the steps 302-308 embody a cycle that may be repeated any number of times, for example until an exception is detected.

In some embodiments, once the apparatus 200 determines that an exception indicator indicates an exception, real-time health data continues to be received and immediately transferred to the data management system 106. In this regard, each subsequently-received real-time health data may be continuously transferred in real-time as it is received via the apparatus 200. In some embodiments, such continuous transfer continues until a termination request is received, for example from the data management system 106. The data management system 106 may generate and/or transmit the termination request to cause the apparatus 200 to terminate the continuous transfer in real-time of each portion of subsequently-received real-time health data.

In a circumstance where an exception is indicated, for example by determining an exception indicator representing an exception, the apparatus 200 at step 310 triggers transfer of locally stored health data to the data management system 106. Specifically, in some embodiments, the apparatus 200 retrieves stored health data from the local datastore 350 for transfer. In some embodiments, the apparatus 200 retrieves a complete stored health data set from the local datastore 350, for example that includes all health data stored via the local datastore 350 (e.g., which may include the most recently received real-time health data). Alternatively or additionally, in some embodiments, the apparatus 200 retrieves at least a portion of stored health data. In some embodiments for example, the apparatus 200 retrieves a portion of stored health data that is within a particular timestamp interval from the current timestamp, for example health data associated with the past hour, day, week, and/or the like.

In some embodiments, the apparatus 200 transfers the locally stored health data via a wired connection with the data management system 106 and/or a wireless connection with the data management system 106. For example, in some embodiments, the data management system 106 embodies a cloud system communicable with via the apparatus 200. The apparatus 200 may transfer the health data to the data management system 106 to cause the data management system 106 to store and/or process such data.

At step 312 the data management system 106 stores the received health data to a long-term storage. As illustrated for example, the data management system 106 stores the received health data to the long-term datastore 352. In some embodiments, the long-term datastore 352 embodies one or more data repositories in or local to the data management system 106. Alternatively or additionally, in some embodiments, the long-term datastore 352 includes one or more remote repositories accessible via the data management system 106, such as a cloud-based database platform. In some embodiments, each portion of the received health data is stored to the long-term datastore 352 associated with metadata indicating a timestamp at which such a portion was captured, a timestamp at which such health data was received by the data management system 106, a user identifier identifying the user associated with the portion of health data, a device identifier identifying the health monitor utilized to capture the portion of health data, and/or the like. It will be appreciated that in some embodiments, the long-term datastore 352 may permanently store health data that is received, and in other embodiments may store such health data for a limited period of time, until deleted by a user, and/or until deleted based on one or more data-driven deletion condition(s).

In some embodiments, the data management system 106 is usable to retrieve, view, and/or otherwise interact with the health data stored to the long-term storage. For example, in some embodiments, a user, administrator, or the like (e.g., a physician in the context of a medical patient being monitored for purposes of providing health services) may login via the data management system 106 to view the health data stored to the long-term storage for one or more entities associated with said user. Additionally or alternatively, in some embodiments, a user, administrator, or the like accesses the health data from the long-term storage to annotate and/or otherwise process such data. It will be appreciated that the long-term datastore 352 may be accessed at any number of times subsequent to the storage of such health data for any of these purposes.

At optional step 314, the data management system 106 processes the health data. In some embodiments, the data management system 106 processes the health data made available via the long-term datastore 352, and/or otherwise received from the apparatus 200.

In some embodiments, the data management system 106 processes the health data to generate a particular health determination. In some embodiments, the health determination represents whether the health data indicates a concerning health condition associated with an entity. Alternatively or additionally, in some embodiments, the health determination represents whether the health data indicates further processing is necessary, for example based on falling outside of an expected range.

Alternatively or additionally, in some embodiments, the data management system 106 processes the health data to apply annotations to at least a portion of the health data. For example, in some embodiments, the annotation data is received in response to user input indicating the annotation for particular portion(s) of the health data. In some embodiments, the data management system 106 stores annotation data linked to corresponding portion(s) of health data selected by the user.

Additionally or alternatively, in some embodiments, the data management system 106 generates one or more exception rule(s) based at least in part on the health data. Additionally or alternatively, in some embodiments, the data management system 106 trains at least one health rules generation model based at least in part on the health data, and/or based at least in part on annotated health data embodying the health data with corresponding annotation data. Additionally or alternatively, in some embodiments, the data management system 106 processes the health data to generate one or more data value(s) for configurable parameter(s) of a health monitor for further use.

At optional step 316, the data management system 106 updates a configuration of a health monitor. In some embodiments, the data management system 106 transmits data to the apparatus 200 for use in altering at least one configurable parameter of the health monitor. In some embodiments, the data management system 106 transmits data embodying at least one generated, updated, and/or retrained health rules generation model for storage and use via the apparatus 200. Alternatively or additionally, in some embodiments, the data management system 106 transmits data embodying exception rule(s) for storage and/or use via the apparatus 200. Additionally or alternatively, in some embodiments, the data management system 106 transmits annotated health data for storage and/or use by the apparatus 200, for example in generating and/or re-training a health rules generation model. In this regard, the data flow in some such embodiments represents an iterative process that maintains an up-to-date configuration of the health monitor as subsequent data is captured via that same health monitor.

FIG. 4 illustrates an example sequence diagram between computing devices for transferring health data based at least in part on a time threshold in accordance with at least one example embodiment of the present disclosure. Specifically, FIG. 4 illustrates a data flow between apparatus 200 and an external data management system 106 for transfer of health data in a circumstance where a time threshold is satisfied, and continuing collection where the time threshold is not satisfied. In some embodiments, the data flow depicted with respect to FIG. 4 is executed in parallel with the data flow depicted and described with respect to FIG. 3. In this regard, the apparatus 200 for example may continually process data to determine exception indicators and simultaneously determine whether a time threshold is satisfied, such that data is transferred in a circumstance where either condition is determined as met.

As illustrated, at step 402 the apparatus 200 receives real-time health data. In some embodiments, the apparatus 200 captures the real-time health data. In some embodiments, the apparatus 200 receives the real-time health data as transmitted from a health monitor. The real-time health data may include any number of health parameters.

At step 404, the apparatus 200 stores the real-time health data to a local storage. As illustrated, the apparatus 200 stores the health data to local datastore 350. In some embodiments, the local datastore 350 is embodied as a subcomponent or subsystem of the apparatus 200. In some embodiments, the local datastore 350 is embodied as an external component or system local to and/or communicable with the apparatus 200. In some embodiments, the apparatus 200 stores the data in a circumstance where a time threshold is not satisfied, for example at step 406.

At step 406, the apparatus 200 determines if a time threshold is satisfied. The apparatus 200 may compare a timestamp interval with a time threshold to perform such a determination. In some embodiments, the apparatus 200 tracks a timestamp interval associated with or otherwise representing a time since last data transfer. The time since last data transfer may represent a length of time since any last data transfer-which may be a transfer of data due to an exception indicated or a transfer due to satisfaction of a time threshold. Alternatively or additionally, in some embodiments, the apparatus 200 tracks a timestamp interval associated with or otherwise representing a time since last default data transfer. The time since last default data transfer may represent a length of time since default health data was last transferred in response to satisfaction of a time threshold. In this regard, the apparatus 200 may transfer at least a portion of health data in a particular cycle, for example every X minutes, hours, days, and/or the like. Additionally or alternatively, in some embodiments, the apparatus 200 may reset this tracked timer in a circumstance where an exception is detected.

In a circumstance where the time threshold is not satisfied, for example by the apparatus 200 determining that the time threshold does not exceed a time threshold, the apparatus 200 at step 408 continues receiving real-time health data. In this regard, the apparatus 200 may return to step 402 to continue receiving new portion(s) of real-time health data. It will be appreciated that in this regard, the steps 402-408 embody a cycle that may be repeated any number of times, for example until an exception is detected.

In a circumstance where an exception is indicated, for example by determining an exception indicator representing an exception, the apparatus 200 at step 410 determines default health data for storage. In some embodiments, the default health data comprises a complete stored health data set stored via a local data storage, for example local datastore 350. In some embodiments, the default health data embodies a complete stored health data set, which include all portions of health data stored to the local datastore 350, for example over a historical period of time. Alternatively or additionally, in some embodiments, the default health data comprises a portion of the complete stored health data set, for example all portion(s) of stored health data associated with a particular most-recent timestamp interval (e.g., health data collected over the last day, week, and/or the like). Additionally or alternatively still, in some embodiments, the default health data includes only the real-time health data. Additionally or alternatively still, in some embodiments, the default health data includes one or more data value(s) derived from the real-time health data and/or stored health data. For example, in some embodiments, the default health data includes an average of data values within the complete health data set stored over a particular time interval.

At step 412, the apparatus 200 triggers transfer of the default health data to the data management system 106. Specifically, in some embodiments, the apparatus 200 retrieves the default health data from the local datastore 350 for transfer. In some embodiments, the apparatus 200 transfers the default health data via a wired connection with the data management system 106 and/or a wireless connection with the data management system 106. For example, in some embodiments, the data management system 106 embodies a cloud system communicable with via the apparatus 200. The apparatus 200 may transfer the default health data to the data management system 106 to cause the data management system 106 to store and/or process such data. In this regard, the data management system 106 may receive and store a record of health data even in circumstances where an exception is not detected for a particular period of time.

At step 414 the data management system 106 stores the received health data to a long-term storage. As illustrated for example, the data management system 106 stores the received default health data to the long-term datastore 352. In some embodiments, the long-term datastore 352 embodies one or more data repositories in or local to the data management system 106. Alternatively or additionally, in some embodiments, the long-term datastore 352 includes one or more remote repositories accessible via the data management system 106, such as a cloud-based database platform. In some embodiments, each portion of the received default health data is stored to the long-term datastore 352 associated with metadata indicating a timestamp at which such a portion was captured, a timestamp at which such health data was received by the data management system 106, a user identifier identifying the user associated with the portion of health data, a device identifier identifying the health monitor utilized to capture the portion of health data, and/or the like. It will be appreciated that in some embodiments, the long-term datastore 352 may permanently store the default health data that is received, and in other embodiments may store such default health data for a limited period of time, until deleted by a user, and/or until deleted based on one or more data-driven deletion condition(s).

In some embodiments, the data management system 106 is usable to retrieve, view, and/or otherwise interact with the health data stored to the long-term storage. For example, in some embodiments, a user, administrator, or the like (e.g., a physician in the context of a medical patient being monitored for purposes of providing health services) may login via the data management system 106 to view the health data stored to the long-term storage for one or more entities associated with said user. Additionally or alternatively, in some embodiments, a user, administrator, or the like accesses the health data from the long-term storage to annotate and/or otherwise process such data. It will be appreciated that the long-term datastore 352 may be accessed at any number of times subsequent to the storage of such health data for any of these purposes. Alternatively or additionally, in some embodiments, the health data stored to the long-term datastore 352 may serve as an extended record of health data for a particular entity across time.

At optional step 416, the data management system 106 processes the health data. In some embodiments, the data management system 106 processes the health data made available via the long-term datastore 352, and/or otherwise received from the apparatus 200.

In some embodiments, the data management system 106 processes the health data to generate a particular health determination. In some embodiments, the health determination represents whether the health data indicates a concerning health condition associated with an entity. Alternatively or additionally, in some embodiments, the health determination represents whether the health data indicates further processing is necessary, for example based on falling outside of an expected range.

Alternatively or additionally, in some embodiments, the data management system 106 processes the health data to apply annotations to at least a portion of the health data. For example, in some embodiments, the annotation data is received in response to user input indicating the annotation for particular portion(s) of the health data. In some embodiments, the data management system 106 stores annotation data linked to corresponding portion(s) of health data selected by the user.

Additionally or alternatively, in some embodiments, the data management system 106 generates one or more exception rule(s) based at least in part on the health data. Additionally or alternatively, in some embodiments, the data management system 106 trains at least one health rules generation model based at least in part on the health data, and/or based at least in part on annotated health data embodying the health data with corresponding annotation data. Additionally or alternatively, in some embodiments, the data management system 106 processes the health data to generate one or more data value(s) for configurable parameter(s) of a health monitor for further use.

At optional step 418, the data management system 106 updates a configuration of a health monitor. In some embodiments, the data management system 106 transmits data to the apparatus 200 for use in altering at least one configurable parameter of the health monitor. In some embodiments, the data management system 106 transmits data embodying at least one generated, updated, and/or retrained health rules generation model for storage and use via the apparatus 200. Alternatively or additionally, in some embodiments, the data management system 106 transmits data embodying exception rule(s) for storage and/or use via the apparatus 200. Additionally or alternatively, in some embodiments, the data management system 106 transmits annotated health data for storage and/or use by the apparatus 200, for example in generating and/or re-training a health rules generation model. In this regard, the data flow (alone or in combination with the data flow depicted and described with respect to FIG. 3 for example) in some such embodiments represents an iterative process that maintains an up-to-date configuration of the health monitor as subsequent data is captured via that same health monitor.

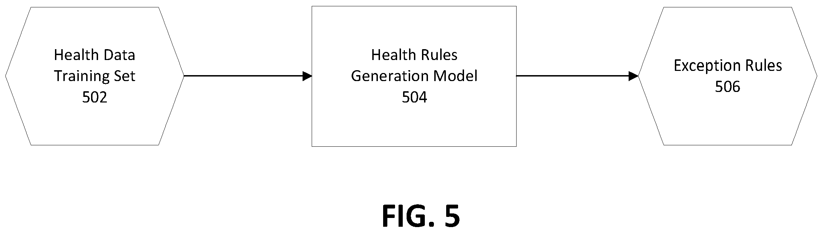

FIG. 5 illustrates an example data flow diagram for generation of exception rules in accordance with at least one example embodiment of the present disclosure. Specifically, FIG. 5 illustrates an example data flow for generation of one or more exception rule(s) via a health-rules generation model. In some embodiments, the data flow as depicted and described is performed by the apparatus 200. In some embodiments, the data flow is performed by an external system-for example a data management system 106-that communicates with the health monitor and/or apparatus 200.

The data flow utilizes a health rules generation model 504. The health rules generation model 504 generates one or more exception rule(s), such as the exception rules 506. In some embodiments, the health rules generation model 504 includes one or more specially configured algorithmic, statistical, machine learning, and/or artificial intelligence model(s). The health rules generation model 504 may be configurable, updatable, and/or otherwise trainable based at least in part on particular data utilized for such purposes. In some embodiments, the health rules generation model 504 includes one or more sub-model(s) that operate in conjunction.

In some embodiments, the health rules generation model 504 is specially configured based at least in part on a health data training set 502. In some embodiments, the health data training set 502 includes a previously collected set of health data, for example embodying a complete health data set collected via one or more health monitor(s). The health data training set 502 may include data value(s) for one or more health parameter(s) represented in such collected health data.

In some embodiments, the health data training set 502 includes one or more portions of annotated health data. Each portion of annotated health data may include the previously collected health data together with annotation data that indicates whether such health data, alone or in combination with one or more other portion(s) of health data, indicates an exception. In some embodiments, the health data training set 502 is generated based at least in part on annotation data provided by at least one medical practitioner or other user associated with a particular health monitor and/or the particular entity corresponding to the health monitor.

In some embodiments, the health data training set 502 includes data corresponding to a single entity. For example, the health data training set 502 may include one or more data portion(s) associated with a particular entity identifier that uniquely identifies a particular entity, such as one particular user of a health monitor (e.g., a patient in a hospital context). In this regard, the health data training set 502 in some embodiments includes annotated health data particular to that entity and/or health monitor, such that the generated exception rules 506 may similarly be particular to that entity and/or health monitor. It should be appreciated that different entities may be associated with different exception rules, where the particular exception rules for a particular entity or health monitor indicate the health data that is an exception for that particular entity and/or health monitor. Such particular exception rules may enable more accurate determinations of an exception for that particular entity and/or health monitor, for example where a non-concerning, normal, or otherwise expected range of data values for particular health parameters for a first entity differs from a non-concerning, normal, or otherwise expected range of data values for the particular health parameters for a second entity. In some embodiments, the health data training set 502 includes data associated with one or more entities and/or health monitors. For example, in some embodiments, the health data training set 502 includes data for a plurality of entities that share one or more characteristics (e.g., biographical characteristics, medical conditions, and/or the like). In other embodiments, the health data training set 502 may include health data and/or annotated health data for all entities and/or health monitors.

In some embodiments, the health rules generation model 504 is specially configured to generate the exception rules 506. The exception rules 506 in some embodiments define normal or otherwise expected range(s) for data value(s) of one or more health parameter(s) represented in the health data utilized to configure and/or otherwise train such health rules generation model 504. The exception rules 506 are defined with respect to all health parameter(s) represented in the health data training set 502, a portion of the health parameter(s) represented in the health data training set 502, and/or a single health parameter represented in the health data training set 502. Alternatively or additionally, in some embodiments, the exception rules 506 includes a single exception rule, or a plurality of health rules that each may indicate an exception.

In some embodiments, the exception rules 506 is/are pushed to and/or stored by the apparatus 200 for further use. For example, in some embodiments, the apparatus 200 stores and/or otherwise maintains the exception rules 506 for use in processing real-time health data and determining whether such real-time health data alone or in combination with other previously-stored health data indicates an exception.

In some embodiments, the exception rules 506 are determined corresponding to and/or based at least in part on particular data associated with a particular health monitor, entity, and/or the like. For example, in some embodiments, the exception rules 506 is/are based at least in part on biographical data corresponding to the entity monitored by the health monitor, for example embodied by and/or associated with the apparatus 200. Such data may include an entity identifier, age, sex, height, weight, and/or the like. Alternatively or additionally, in some embodiments, the exception rules 506 is/are based at least in part on a health monitor type, type of health parameter(s) being monitored, and/or the like.

In some embodiments, the exception rules 506 generate an exception indicator that includes a binary value, for example a first value indicating an exception and a second value indicating no exception. Alternatively in some embodiments, the exception rules 506 generates an exception indicator that determines between a plurality of different level(s) and/or gradation(s) of exception. For example, in some embodiments, the exception indicator may represent a first value indicating no exception, a second value indicating a first level of exception (e.g., concerning but not critical data value(s)), and/or a third value indicating a second level of exception (e.g., concerning and critical data value(s)). In some embodiments, the exception rules 506 define different range(s) for data value(s) of various health parameter(s) that correspond to the different levels of exception.

In some embodiments, the exception rules 506 indicate different data-driven triggers, each of which may initiate one or more different methods of subsequent processing of captured real-time health data. For example, in some embodiments, the exception rules 506 are utilized to generate an exception indicator representing one of three or more candidate values-a non-concerning exception indicator, a moderately concerning exception indicator, and a critically concerning exception indicator. In some embodiments, transfer of stored health data may not be triggered in circumstances where a non-concerning exception indicator is generated, but may be triggered in circumstances where a moderately or critically concerning exception indicator is generated. Additionally, in some such embodiments, transfer of subsequently-received real-time health data may be triggered only in instances where a critically concerning exception indicator is generated, and not where a moderately concerning exception indicator is generated.

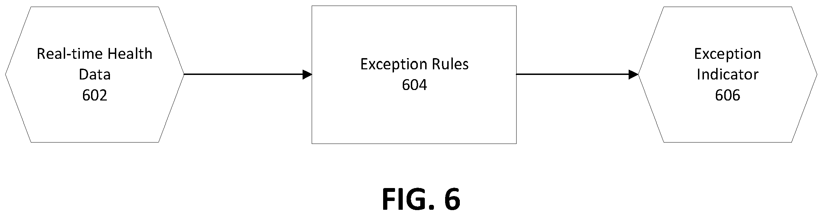

FIG. 6 illustrates an example data flow diagram for determination of an exception indicator in accordance with at least one example embodiment of the present disclosure. Specifically, FIG. 6 illustrates an example data flow for generation of an exception indicator via exception rules. In some embodiments, the data flow as depicted and described is performed by the apparatus 200. In some embodiments, the data flow is performed by a health monitor, for example embodied by, as a subcomponent of, or otherwise communicable with the apparatus 200. In this regard, the real-time health data 602 in some embodiments represents the most recent data values for one or more health parameter(s) monitored via the apparatus 200 and/or an associated health monitor.

As illustrated, real-time health data 602 is applied to the exception rules 604. The exception rules 604 may define particular algorithm(s) for processing the real-time health data 602 to detect whether the real-time health data 602 indicates an exception. In some embodiments, the exception rules 604 define expected and/or non-concerning range(s) for health parameter(s) in the real-time health data 602. For example, in some embodiments, the exception rules 604 include boundary data value(s) defining such range(s), for example maximum data value(s) and/or minimum data value(s) for each of one or more particular health parameter(s) represented in health data.

The exception rules 604 may be previously stored to the apparatus 200. In some embodiments, the apparatus 200 generates the exception rules 604, for example utilizing the health rules generation model(s) as described with respect to FIG. 5. Alternatively or additionally, in some embodiments a data monitoring system generates the exception rules 604, and pushes such exception rules to the apparatus 200 for subsequent use.

The exception rules 604 includes any number of exception rules. In some embodiments, the exception rules 604 includes a single exception rule. The single exception rule may correspond to a single health parameter or a combination of health parameters. In some embodiments, the exception rules 604 includes a plurality of exception rules. In some embodiments, an exception is indicated in a circumstance where any one exception rule of the plurality of exception rules is satisfied. Alternatively or additionally, in some embodiments, an exception is indicated in a circumstance where each exception rule or all of the plurality of exception rules is satisfied.

As described, the exception indicator 606 embodies data representing whether the real-time health data 602 indicates an exception or no exception. For example, in some embodiments, the exception indicator 606 embodies a first particular value in a circumstance where the real-time health data 602 indicates an exception, and a second particular value in a circumstance where the real-time health data 602 indicates no exception. In this regard, the exception indicator 606 may be compared with a predetermined value (e.g., the first particular value) and, if equivalent, is determined to indicate an exception. In one example context, an exception represents data value(s) that indicate a concern to the health of an entity.

It should be appreciated that the data flow depicted and described with respect to FIG. 6 may be performed for any number of portions of real-time health data 602. For example, in some embodiments, the apparatus 200 performs the data flow as a cycle each time new real-time health data 602 is received and/or captured. The apparatus 200 may thus determine whether each subsequently captured portion of real-time health data 602 indicates an exception.

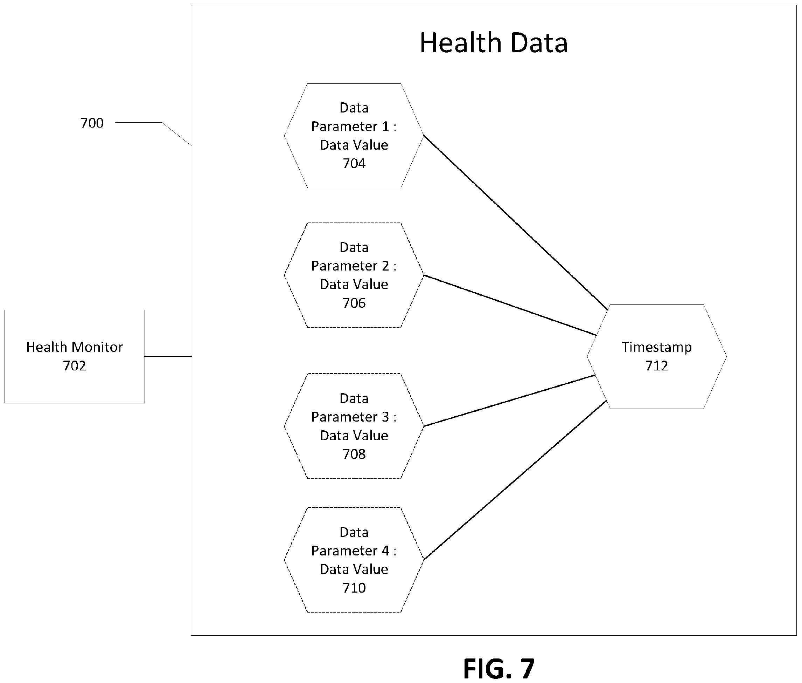

FIG. 7 illustrates an example data architecture for health data in accordance with at least one example embodiment of the present disclosure. Specifically, FIG. 7 illustrates an example data architecture for health data 700. The health data 700 in some embodiments represents an example of real-time health data capturable by a particular device, for example a health monitor 702. The health monitor 702 may be embodied by, include, or be associated with an apparatus 200.

As illustrated, the health data 700 includes data values corresponding to a plurality of health parameters. The health data 700 includes a data value for a first data parameter 704. In some embodiments, the data value for the first data parameter 704 represents any value within a possible range of data values for a first health parameter tracked by the health monitor 702. Additionally, in some embodiments, the health data 700 may include any number of additional data value(s) corresponding to various health parameter(s) of the health data 700. As illustrated, the health data 700 optionally includes a data value corresponding to a second data parameter 706, a data value corresponding to a third data parameter, and a data value corresponding to a fourth data parameter 708. In this regard, in some embodiments each data value corresponds to a data parameter representing a different health parameter. In some embodiments, the health monitor 702 includes various sensor(s), probe(s), and/or sub-monitor(s) that capture the data value for each independent health parameter. It will be appreciated that the data value for each particular health parameter may fall within a different range corresponding to the particular health parameter.

The health data 700 further includes a timestamp 712. In some embodiments, the timestamp 712 represents a particular time at which such data values for the various health parameters were captured. For example, in some embodiments the health monitor 702, and/or an associated monitor-linked support system, tracks a current timestamp representing the current time. The value of such current timestamp may be assigned to the timestamp 712 at the time a particular sample of the health data 700 is captured, received, or otherwise collected. The timestamp 712 is associated with each of the data values corresponding to the various data parameters, including data values for the first, second, third, and fourth data parameters 704, 706, 708,and 710 respectively. In this regard, any one of the data values for the particular health parameters may be linked to the timestamp 712 to indicate what the value of the particular health parameter was at a particular time.

In some embodiments, each portion of health data includes data values corresponding to the same health parameters. In other embodiments, different portions of health data may include data values for one or more different health parameters. For example, in some embodiments, a portion of health data includes data value(s) corresponding to one or more alternative health parameter(s), one or more additional health parameter(s), and/or one or more removed health parameter(s) removed from the health data.

### EXAMPLE PROCESSES OF THE DISCLOSURE

Having described example systems and apparatuses, related data flows, and data architectures in accordance with the disclosure, example processes of the disclosure will now be discussed. It will be appreciated that each of the flowcharts depicts an example computer-implemented process that is performable by one or more of the apparatuses, systems, devices, and/or computer program products described herein, for example utilizing one or more of the specially configured components thereof.

The blocks indicate operations of each process. Such operations may be performed in any of a number of ways, including, without limitation, in the order and manner as depicted and described herein. In some embodiments, one or more blocks of any of the processes described herein occur in-between one or more blocks of another process, before one or more blocks of another process, in parallel with one or more blocks of another process, and/or as a sub-process of a second process. Additionally or alternatively, any of the processes in various embodiments include some or all operational steps described and/or depicted, including one or more optional blocks in some embodiments. With regard to the flowcharts illustrated herein, one or more of the depicted block(s) in some embodiments is/are optional in some, or all, embodiments of the disclosure. Optional blocks are depicted with broken (or "dashed") lines. Similarly, it should be appreciated that one or more of the operations of each flowchart may be combinable, replaceable, and/or otherwise altered as described herein.

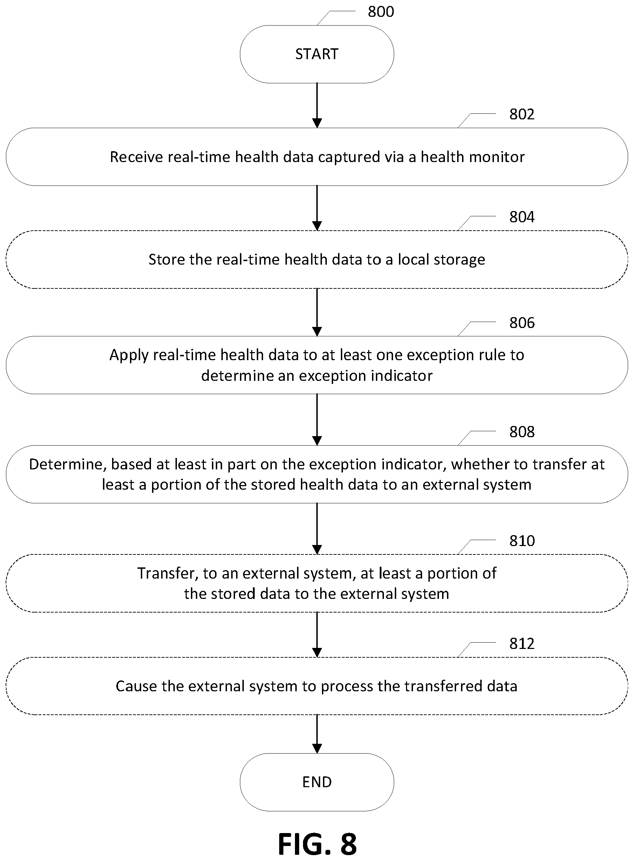

FIG. 8 illustrates a flowchart including example operations of an example computer-implemented process for managing storage of health data in accordance with at least one example embodiment of the present disclosure. In some embodiments, the process 800 is embodied by computer program code stored on a non-transitory computer-readable storage medium of a computer program product configured for execution to perform the process as depicted and described. Alternatively or additionally, in some embodiments, the process 800 is performed by one or more specially configured computing devices, such as the apparatus 200 alone or in communication with one or more other component(s), device(s), system(s), and/or the like. In this regard, in some such embodiments, the apparatus 200 is specially configured by computer-coded instructions (e.g., computer program instructions) stored thereon, for example in the memory 204 and/or another component depicted and/or described herein and/or otherwise accessible to the apparatus 200, for performing the operations as depicted and described. In some embodiments, the apparatus 200 is in communication with one or more external apparatus(es), system(s), device(s), and/or the like, to perform one or more of the operations as depicted and described. For example, the apparatus 200 in some embodiments is in communication with a separate health monitor, data management system, monitor-linked support system, monitor configuration system, and/or the like. For purposes of simplifying the description, the process 800 is described as performed by and from the perspective of the apparatus 200.

The process 800 begins at operation 802. At operation 802, the apparatus 200 includes data monitoring circuitry 210, rule maintenance circuitry 212, smart data transfer & storage circuitry 214, communications circuitry 208, input/output circuitry 206, processor 202, and/or a combination thereof, that receives real-time health data captured via a health monitor. The real-time health data may represent data value(s) for particular health parameter(s) associated with a current timestamp. In some embodiments, the health monitor is embodied as a subcomponent of the apparatus 200. In some embodiments, the health monitor is external from the apparatus 200.

At optional operation 804, the apparatus 200 includes data monitoring circuitry 210, rule maintenance circuitry 212, smart data transfer & storage circuitry 214, communications circuitry 208, input/output circuitry 206, processor 202, and/or a combination thereof, that stores the real-time health data to a local storage. In some embodiments, the local storage embodies a local datastore maintained by the apparatus 200. Alternatively or additionally, in some embodiments, the local storage embodies a datastore maintained by a monitor-linked support system communicable with the apparatus 200. In any such embodiments, the apparatus 200 may store to the local storage in less time than a corresponding non-local long-term data storage, for example maintained by a system remote from the apparatus 200. In some embodiments, the apparatus 200 transmits the real-time health data to a monitor-linked support system over a short-range communications network (e.g., a wire, Bluetooth low energy, and/or the like), where the monitor-linked support system maintains the local storage.

In some embodiments, the local storage is configured having a particular maximum size. In this regard, data may be stored to the local storage without overwriting any data until the maximum size is reached. In a circumstance where the local storage is full, one or more portion(s) of health data stored to the local storage may be overwritten. For example, in some embodiments, the apparatus 200 overwrites data in the local storage specifically by overwriting the data associated with the oldest timestamp in a defined historical time series of stored health data within the local storage.

At operation 806, the apparatus 200 includes data monitoring circuitry 210, rule maintenance circuitry 212, smart data transfer & storage circuitry 214, communications circuitry 208, input/output circuitry 206, processor 202, and/or a combination thereof, that applies the real-time health data to at least one exception rule. The apparatus 200 applies the real-time health data to the at least one exception rule to determine an exception indicator. In some embodiments, the exception indicator embodies data representing whether the real-time health data satisfies one of the at least one exception rules, or in some embodiments all of the at least one exception rules. In some embodiments for example, the at least one exception rule is satisfied in a circumstance where the real-time health data is determined to indicate a health concern. In some embodiments, the at least one exception rule is pushed to the apparatus 200 from another system or device, such as a monitor configuration system. Alternatively or additionally, in some embodiments, the at least one exception rule is generated by the apparatus 200 or an external system utilizing one or more health rules generation model(s) as described herein.

At operation 808, the apparatus 200 includes data monitoring circuitry 210, rule maintenance circuitry 212, smart data transfer & storage circuitry 214, communications circuitry 208, input/output circuitry 206, processor 202, and/or a combination thereof, that determines, based at least in part on the exception indicator, whether to transfer at least a portion of the stored health data to an external system. In some embodiments, the apparatus 200 determines whether to transfer any data to the external system based at least in part on whether the exception indicator indicates an exception. In some such embodiments, the apparatus 200 transfers data in a circumstance where the exception indicator indicates an exception, and does not transfer such data in a circumstance where the exception indicator does not indicate an exception (e.g., indicates no exception). In some embodiments, the apparatus 200 compares the exception indicator to a predetermined value representing an exception. The apparatus 200 may determine that at least the portion of the stored health data should be transferred in a circumstance where the exception indicator is equivalent to the predetermined value, and determines that at least the portion of stored health data should not be transferred in a circumstance where the exception indicator is not equivalent to the predetermined value. It will be appreciated that in other embodiments, the exception indicator may be compared with a different value, for example a predetermined value indicating no exception, to determine whether to transfer at least the portion of the stored health data in alternative circumstances.

At optional operation 810, the apparatus 200 includes data monitoring circuitry 210, rule maintenance circuitry 212, smart data transfer & storage circuitry 214, communications circuitry 208, input/output circuitry 206, processor 202, and/or a combination thereof, that transfers, to the external system, at least a portion of data to the external system. In some embodiments, at least the portion of stored health data includes a complete stored health data set representing all portions of health data stored to a local datastore associated with the apparatus 200 and/or the health monitor. Additionally or alternatively, in some embodiments, the at least the portion of the stored data includes the most recently captured real-time health data received by the apparatus 200. Additionally or alternatively still, in some embodiments, the apparatus 200 retrieves a particular portion of the stored health data that corresponds to a particular timestamp interval (e.g., data over the previous X minutes, hours, days, and/or the like).

At optional operation 812, the apparatus 200 includes data monitoring circuitry 210, rule maintenance circuitry 212, smart data transfer & storage circuitry 214, communications circuitry 208, input/output circuitry 206, processor 202, and/or a combination thereof, that causes the external system to process the transferred data. In some embodiments, the apparatus 200 transmits one or more message(s) including and/or associated with the portion of the stored data transferred to the external system that indicates that such data should be stored and/or processed. In some embodiments, the apparatus 200 transmits one or more message(s) that causes the external system to determine whether the at least the portion of the stored data indicates a concerning health condition, and/or causes the external system to make at least the portion of the stored data transferred to the external system available for viewing, annotating, and/or further processing by a user of the external system (e.g., a medical provider that accesses the transferred health data to determine whether such data indicates a concerning health condition).

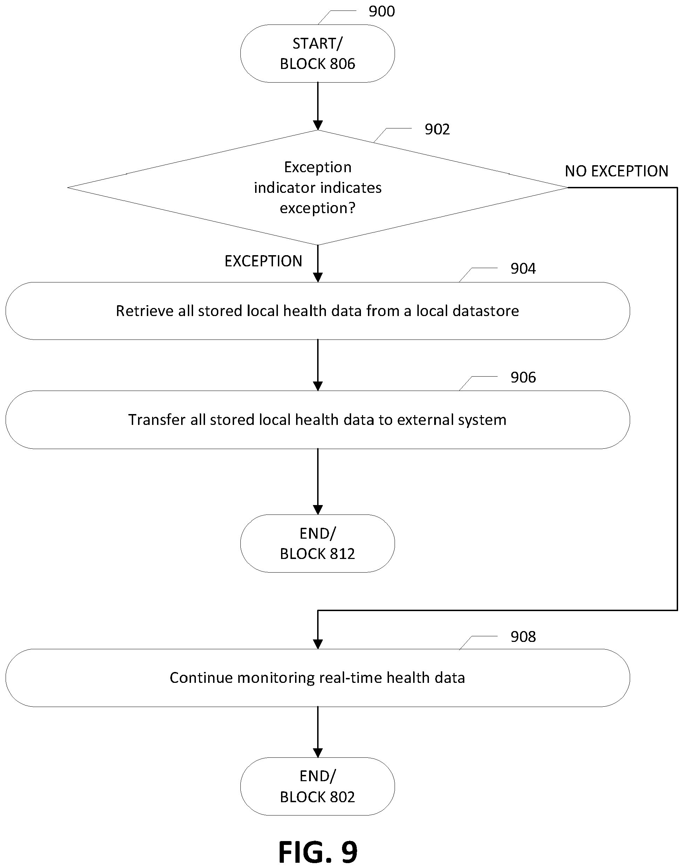

FIG. 9 illustrates a flowchart including example operations of an example computer-implemented process for determining whether to transfer health data based at least in part on an exception indicator in accordance with at least one example embodiment of the present disclosure. In some embodiments, the process 900 is embodied by computer program code stored on a non-transitory computer-readable storage medium of a computer program product configured for execution to perform the process as depicted and described. Alternatively or additionally, in some embodiments, the process 900 is performed by one or more specially configured computing devices, such as the apparatus 200 alone or in communication with one or more other component(s), device(s), system(s), and/or the like. In this regard, in some such embodiments, the apparatus 200 is specially configured by computer-coded instructions (e.g., computer program instructions) stored thereon, for example in the memory 204 and/or another component depicted and/or described herein and/or otherwise accessible to the apparatus 200, for performing the operations as depicted and described. In some embodiments, the apparatus 200 is in communication with one or more external apparatus(es), system(s), device(s), and/or the like, to perform one or more of the operations as depicted and described. For example, the apparatus 200 in some embodiments is in communication with a separate health monitor, data management system, monitor-linked support system, monitor configuration system, and/or the like. For purposes of simplifying the description, the process 900 is described as performed by and from the perspective of the apparatus 200.

The process 900 begins at operation 902. In some embodiments, the process 900 begins after one or more operations depicted and/or described with respect to any one of the other processes described herein. For example, in some embodiments as depicted, the process 900 begins after execution of operation 806. In this regard, some or all of the process 900 may replace or supplement one or more blocks depicted and/or described with respect to any of the processes described herein. Upon completion of the process 900, the flow of operations may terminate. Additionally or alternatively, as depicted, upon completion of the process 900 in some embodiments, flow may return to one or more operation(s) of another process, such as the operation 812 or 802 as depicted and described. It will be appreciated that, in some embodiments, the process 900 embodies a sub-process of one or more other process(es) depicted and/or described herein, for example the process 800.

At operation 902, the apparatus 200 includes data monitoring circuitry 210, rule maintenance circuitry 212, smart data transfer & storage circuitry 214, communications circuitry 208, input/output circuitry 206, processor 202, and/or a combination thereof, that determines whether the exception indicator indicates an exception. In some embodiments, the apparatus 200 compares the exception indicator to one or more predetermined values, for example a first predetermined value indicating an exception or a second predetermined value indicating no exception, to determine whether the exception indicator indicates exception. In some embodiments, the apparatus 200 determines whether the exception indicator indicates an exception as described with respect to operation 808 herein.

In circumstances where the exception indicator indicates exception, flow proceeds to operation 904. At operation 904, the apparatus 200 includes data monitoring circuitry 210, rule maintenance circuitry 212, smart data transfer & storage circuitry 214, communications circuitry 208, input/output circuitry 206, processor 202, and/or a combination thereof, that retrieves all stored local health data from a local datastore. In some embodiments, the apparatus 200 queries the local datastore for a complete health data set stored by the local datastore, which includes all stored local health data maintained by the local datastore. In some embodiments, the apparatus 200 maintains the local datastore, and retrieves all stored local health data by directly accessing the local datastore (e.g., to query for all such stored local health data).

At operation 906, the apparatus 200 includes data monitoring circuitry 210, rule maintenance circuitry 212, smart data transfer & storage circuitry 214, communications circuitry 208, input/output circuitry 206, processor 202, and/or a combination thereof, that transfers all the stored health data to an external system. In some embodiments, the apparatus 200 transmits one or more message(s) that include all stored local health data retrieved from the local datastore, for example as described with respect to operation 904. The apparatus 200 may transfer all stored local health data to the external system to cause the external system to store such data to a long-term datastore maintained by the external system.

In circumstances where the exception indicator does not indicate exception, flow proceeds to operation 908. At operation 908, the apparatus 200 includes data monitoring circuitry 210, rule maintenance circuitry 212, smart data transfer & storage circuitry 214, communications circuitry 208, input/output circuitry 206, processor 202, and/or a combination thereof, that continues monitoring real-time health data. It will be appreciated that such operations ay form a loop, such that data is continually monitored until the exception indicator indicates an exception, for example. In this regard, the apparatus 200 may return to operation 802 as depicted and described herein.

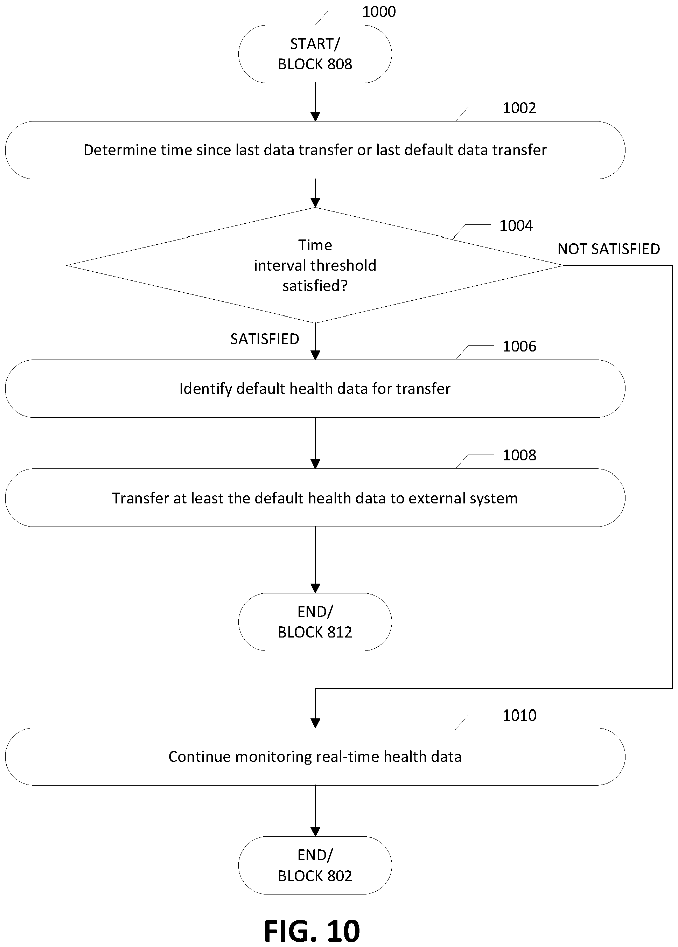

FIG. 10 illustrates a flowchart including example operations of an example computer-implemented method for determining whether to transfer health data based at least in part on a time interval threshold in accordance with at least one example embodiment of the present disclosure. In some embodiments, the process 1000 is embodied by computer program code stored on a non-transitory computer-readable storage medium of a computer program product configured for execution to perform the process as depicted and described. Alternatively or additionally, in some embodiments, the process 1000 is performed by one or more specially configured computing devices, such as the apparatus 200 alone or in communication with one or more other component(s), device(s), system(s), and/or the like. In this regard, in some such embodiments, the apparatus 200 is specially configured by computer-coded instructions (e.g., computer program instructions) stored thereon, for example in the memory 204 and/or another component depicted and/or described herein and/or otherwise accessible to the apparatus 200, for performing the operations as depicted and described. In some embodiments, the apparatus 200 is in communication with one or more external apparatus(es), system(s), device(s), and/or the like, to perform one or more of the operations as depicted and described. For example, the apparatus 200 in some embodiments is in communication with a separate health monitor, data management system, monitor-linked support system, monitor configuration system, and/or the like. For purposes of simplifying the description, the process 1000 is described as performed by and from the perspective of the apparatus 200.

The process 1000 begins at operation 1002. In some embodiments, the process 1000 begins after one or more operations depicted and/or described with respect to any one of the other processes described herein. For example, in some embodiments as depicted, the process 1000 begins after execution of operation 806. In this regard, some or all of the process 1000 may replace or supplement one or more blocks depicted and/or described with respect to any of the processes described herein. Upon completion of the process 1000, the flow of operations may terminate. Additionally or alternatively, as depicted, upon completion of the process 1000 in some embodiments, flow may return to one or more operation(s) of another process, such as the operation 812 or 802 as depicted and described. It will be appreciated that, in some embodiments, the process 1000 embodies a sub-process of one or more other process(es) depicted and/or described herein, for example the process 800.

At operation 1002, the apparatus 200 includes data monitoring circuitry 210, rule maintenance circuitry 212, smart data transfer & storage circuitry 214, communications circuitry 208, input/output circuitry 206, processor 202, and/or a combination thereof, that determines a time since last data transfer or a last default data transfer. In some embodiments, the apparatus 200 tracks one or more data value(s) embodying a time since last data interval or a last default data transfer. Data embodying the time since last data transfer may be determined, for example, embodying a time interval since a timestamp at which any data transfer was performed-whether triggered based at least in part on an indicated exception or a time interval threshold. Alternatively or additionally, in some embodiments the apparatus 200 tracks a data interval since a last data transferred triggered based at least in part on a detected exception specifically. In some embodiments, the apparatus 200 maintains a separate time interval representing the time since last default data transfer based at least in part on a timestamp at which a last data transfer triggered based at least in part on a time interval threshold was initiated. It should be appreciated that the apparatus 200 may track one of such timestamp intervals, or in some embodiments may track both such timestamp intervals separately (e.g., a first timestamp interval representing a time since last data transfer and a second timestamp interval representing a time since a last default data transfer).

At operation 1004, the apparatus 200 includes data monitoring circuitry 210, rule maintenance circuitry 212, smart data transfer & storage circuitry 214, communications circuitry 208, input/output circuitry 206, processor 202, and/or a combination thereof, that determines whether a time interval threshold is satisfied. For example, in some embodiments, the apparatus 200 compares at least one time interval with a determined time interval threshold. The time interval threshold in some embodiments represents a predetermined value representing a maximum amount of time between transfer of at least a portion of health data from the apparatus 200 to the external system. In some embodiments, the time interval threshold is set by a user of the apparatus 200, and/or a user, administrator, or the like having access to the external system.

In some embodiments, the apparatus 200 compares a time interval representing the time since last data transfer or the time since last default data transfer to the time interval threshold. In some embodiments, the apparatus 200 determines that the time interval threshold is satisfied in a circumstance where the time interval is equal to and/or exceeds the time interval threshold. In other embodiments, the apparatus 200 determines that the time interval threshold is satisfied in a circumstance where the time interval is equal to or less than the time interval threshold, or otherwise equivalent to a particular value represented by the time interval threshold (e.g., zero in a circumstance where the time interval is decremented until it reaches zero).

In some embodiments, flow continues to operation 1006 in a circumstance where the time interval threshold is satisfied. At operation 1006, the apparatus 200 includes data monitoring circuitry 210, rule maintenance circuitry 212, smart data transfer & storage circuitry 214, communications circuitry 208, input/output circuitry 206, processor 202, and/or a combination thereof, that identifies at least default health data for transfer. For example, in some embodiments, the apparatus 200 identifies default health data including the most recent portion of data received via the apparatus 200. For example, the default health data in some embodiments embodies the most-recent real-time health data received by the apparatus 200. In other embodiments, the default health data includes stored health data associated with a particular recent timestamp interval, for example stored associated with a particular previous particular number of minute(s), hour(s), day(s), and/or the like. Alternatively or additionally, in some embodiments, the apparatus 200 identifies default health data including stored health data associated with particular timestamps, for example stored health data at each hour mark, day, and/or the like, stored to a local datastore for the apparatus 200.

In some embodiments, default health data includes one or more data value(s) processed from stored health data. For example, in some embodiments, the default health data includes an averaged value for each health parameter represented in each portion of health data stored to the local datastore. Alternatively or additionally, in some embodiments, the default health data includes a minimum and/or maximum value for one or more particular health parameter(s) represented in each portion of health data stored to the local datastore.

At operation 1008, the apparatus 200 includes data monitoring circuitry 210, rule maintenance circuitry 212, smart data transfer & storage circuitry 214, communications circuitry 208, input/output circuitry 206, processor 202, and/or a combination thereof, that transfers at least the default health data to an external system. In some embodiments, the apparatus 200 transmits one or more message(s) that includes the default health data. The apparatus 200 may transfer the default health data to cause the external system to store such data to a long-term datastore maintained by the external system. In some embodiments, the external system stores the default health data associated with a timestamp when such data is received.

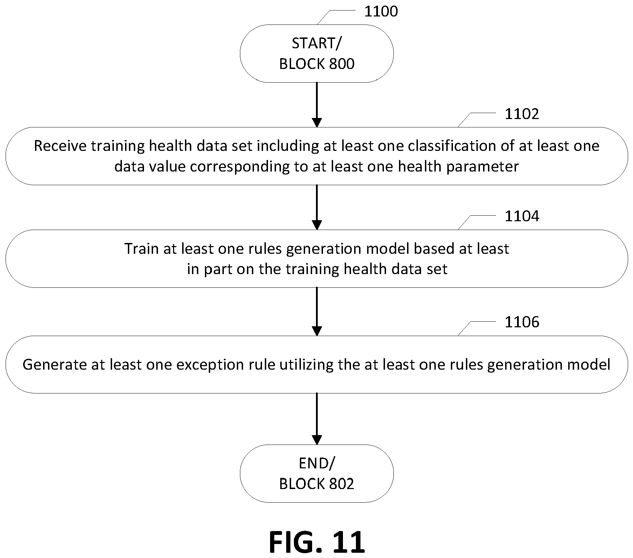

FIG. 11 illustrates a flowchart including example operations of an example computer-implemented method for generating at least one exception rule in accordance with at least one example embodiment of the present disclosure. In some embodiments, the process 1100 is embodied by computer program code stored on a non-transitory computer-readable storage medium of a computer program product configured for execution to perform the process as depicted and described. Alternatively or additionally, in some embodiments, the process 1100 is performed by one or more specially configured computing devices, such as the apparatus 200 alone or in communication with one or more other component(s), device(s), system(s), and/or the like. In this regard, in some such embodiments, the apparatus 200 is specially configured by computer-coded instructions (e.g., computer program instructions) stored thereon, for example in the memory 204 and/or another component depicted and/or described herein and/or otherwise accessible to the apparatus 200, for performing the operations as depicted and described. In some embodiments, the apparatus 200 is in communication with one or more external apparatus(es), system(s), device(s), and/or the like, to perform one or more of the operations as depicted and described. For example, the apparatus 200 in some embodiments is in communication with a separate health monitor, data management system, monitor-linked support system, monitor configuration system, and/or the like. For purposes of simplifying the description, the process 1100 is described as performed by and from the perspective of the apparatus 200.

The process 1100 begins at operation 1102. In some embodiments, the process 1100 begins after one or more operations depicted and/or described with respect to any one of the other processes described herein. For example, in some embodiments as depicted, the process 1100 begins at the beginning of process 800. In this regard, some or all of the process 1100 may replace or supplement one or more blocks depicted and/or described with respect to any of the processes described herein. Upon completion of the process 1100, the flow of operations may terminate. Additionally or alternatively, as depicted, upon completion of the process 1100 in some embodiments, flow may return to one or more operation(s) of another process, such as the operation 802 as depicted and described. It will be appreciated that, in some embodiments, the process 1100 embodies a sub-process of one or more other process(es) depicted and/or described herein, for example the process 800.

At operation 1102, the apparatus 200 includes data monitoring circuitry 210, rule maintenance circuitry 212, smart data transfer & storage circuitry 214, communications circuitry 208, input/output circuitry 206, processor 202, and/or a combination thereof, that receives training health data set. In some embodiments, the training health data set includes one or more portion(s) of training health data. In some embodiments, each portion of training health data includes at least one classification of at least one data value corresponding to at least one health parameter represented in a portion of health data. For example, in some embodiments, the training health data includes at least one classification indicating whether one or more data value(s) corresponding to one or more health parameter(s) indicates an exception or does not indicate an exception. In some embodiments, each portion of training health data embodies annotated health data, wherein the annotation data corresponding to each portion of annotated health data represents the at least one classification. The annotation data in some embodiments is generated via another user associated with an external system, for example a health practitioner interacting with a data management system. In some embodiments, the at least one classification embodies an indication of whether one or more data value(s) corresponding to a single health parameter or a combination of health parameter(s) indicates a concerning health condition or a non-concerning health condition. In some embodiments, the at least one classification includes a first classification embodying a first value of an exception indicator indicating an exception, and a second classification embodying a second value of an exception indicator indicating no exception. In some embodiments, the at least one classification corresponds to any one of a plurality of candidate values for an exception indicator.

At operation 1104, the apparatus 200 includes data monitoring circuitry 210, rule maintenance circuitry 212, smart data transfer & storage circuitry 214, communications circuitry 208, input/output circuitry 206, processor 202, and/or a combination thereof, that trains at least one rules generation model based at least in part on the training health data set. The at least one rules generation model is trained to identify differences between at least a first classification and a second classification. For example, in some embodiments, the at least one rules generation model is trained to generate data indicating a most likely classification based at least in part on the training health data set, and/or generate probability data indicating a likelihood that particular health data represents each classification of a plurality of candidate classifications. In this regard, the at least one rules generation model may be trained to learn or otherwise determine data trend(s), pattern(s), and/or indicator(s) that indicate each particular classification of a plurality of candidate classifications. In some embodiments, the at least one rules generation model embodies or includes at least one algorithmic, statistical, machine learning, and/or artificial intelligence model. It should be appreciated that any of a myriad of known and/or custom training methodologies may be utilized to train the at least one rules generation model based at least in part on the training health data set.

At operation 1106, the apparatus 200 includes data monitoring circuitry 210, rule maintenance circuitry 212, smart data transfer & storage circuitry 214, communications circuitry 208, input/output circuitry 206, processor 202, and/or a combination thereof, that generates at least one exception rule utilizing the at least one rules generation model. In some embodiments, the at least one rules generation model generates at least one exception rule that is determined to indicate an exception based at least in part on the training health data set. For example, in some embodiments, the training health data set includes annotation data indicating whether a portion of annotated health data in the training health data set indicates an exception, such that during training the at least one rules generation model may identify the data value(s), data pattern(s), and/or trend(s) that indicate an exception. The at least one exception rule may be generated as output by the at least one rules generation model in response to such training.

In some embodiments, the apparatus 200 stores the exception rule for use in processing real-time health data, for example as depicted and described with respect to FIG. 8. Alternatively or additionally, in some embodiments, the apparatus 200 pushes the at least one exception rule to a health monitor for use in processing real-time health data.

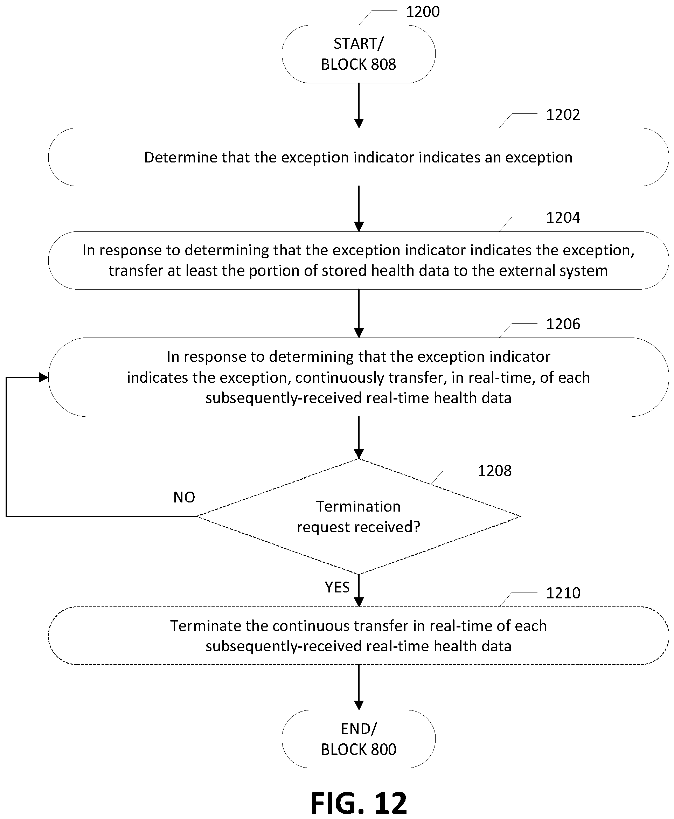

FIG. 12 illustrates a flowchart including example operations of an example computer-implemented method for continuously transferring real-time health data in accordance with at least one example embodiment of the present disclosure. In some embodiments, the process 1200 is embodied by computer program code stored on a non-transitory computer-readable storage medium of a computer program product configured for execution to perform the process as depicted and described. Alternatively or additionally, in some embodiments, the process 1200 is performed by one or more specially configured computing devices, such as the apparatus 200 alone or in communication with one or more other component(s), device(s), system(s), and/or the like. In this regard, in some such embodiments, the apparatus 200 is specially configured by computer-coded instructions (e.g., computer program instructions) stored thereon, for example in the memory 204 and/or another component depicted and/or described herein and/or otherwise accessible to the apparatus 200, for performing the operations as depicted and described. In some embodiments, the apparatus 200 is in communication with one or more external apparatus(es), system(s), device(s), and/or the like, to perform one or more of the operations as depicted and described. For example, the apparatus 200 in some embodiments is in communication with a separate health monitor, data management system, monitor-linked support system, monitor configuration system, and/or the like. For purposes of simplifying the description, the process 1200 is described as performed by and from the perspective of the apparatus 200.

The process 1200 begins at operation 1202. In some embodiments, the process 1200 begins after one or more operations depicted and/or described with respect to any one of the other processes described herein. For example, in some embodiments as depicted, the process 1200 begins after execution of operation 808. In this regard, some or all of the process 1200 may replace or supplement one or more blocks depicted and/or described with respect to any of the processes described herein. Upon completion of the process 1200, the flow of operations may terminate. Additionally or alternatively, as depicted, upon completion of the process 1200 in some embodiments, flow may return to one or more operation(s) of another process, such as the operation 802 as depicted and described. It will be appreciated that, in some embodiments, the process 1200 embodies a sub-process of one or more other process(es) depicted and/or described herein, for example the process 800.

At operation 1202, the apparatus 200 includes data monitoring circuitry 210, rule maintenance circuitry 212, smart data transfer & storage circuitry 214, communications circuitry 208, input/output circuitry 206, processor 202, and/or a combination thereof, that determines that the exception indicator indicates an exception. In some embodiments, the apparatus 200 determines that the exception indicator indicates an exception by comparing the exception indicator with a predetermined value, for example that represents an exception. In some such embodiments, the apparatus 200 determines that the exception indicator indicates an exception in a circumstance where the value of the exception indicator is equivalent to the predetermined value. Alternatively or additionally, in some embodiments the apparatus 200 determines that the exception indicator indicates an exception in a circumstance where any of one or more exception rule(s) outputs data indicating determination that one or more exception rule(s) is satisfied.

At operation 1204, the apparatus 200 includes data monitoring circuitry 210, rule maintenance circuitry 212, smart data transfer & storage circuitry 214, communications circuitry 208, input/output circuitry 206, processor 202, and/or a combination thereof, that, in response to determining that the exception indicator indicates the exception, transfer at least a portion of the stored health data to the external data system. In some embodiments, the apparatus 200 transfers at least the portion of the stored health data as described with respect to operation 810.

At operation 1206, the apparatus 200 includes data monitoring circuitry 210, rule maintenance circuitry 212, smart data transfer & storage circuitry 214, communications circuitry 208, input/output circuitry 206, processor 202, and/or a combination thereof, that in response to determining that the exception indicator indicates the exception, continuously transfer each subsequently-received portion of real-time health data. In some embodiments, the apparatus 200 continuously transfers each portion of subsequently-received real-time health data in real-time as such data is received. In this regard, as each subsequently-received real-time health data is received by the apparatus 200, the apparatus 200 may transmit one or more message(s) to the external system that causes the external system to store the transmitted subsequently-received real-time health data.

In some embodiments, the apparatus 200 continues to continuously transfer subsequently-received real-time health data until a particular data-driven determination is reached. At optional operation 1208, the apparatus 200 includes data monitoring circuitry 210, rule maintenance circuitry 212, smart data transfer & storage circuitry 214, communications circuitry 208, input/output circuitry 206, processor 202, and/or a combination thereof, that determines whether a termination request has been received. For example, in some embodiments, the apparatus 200 monitors for the termination request received in response to user input and/or in response to one or more message(s) transmitted from the external system. In some embodiments, the external system generates and/or transmits the termination request to the apparatus 200 in response to user input (e.g., from a medical professional) that indicates that such subsequently-received real-time health data no longer indicates an exception (e.g., has returned to a normal range). Alternatively or additionally, in some embodiments, the termination request is automatically received and/or otherwise generated by the apparatus 200 after a particular period of time. Alternatively or additionally still, in some embodiments, the termination request is automatically received in a circumstance where an exception is no longer indicated.

At optional operation 1210 the apparatus 200 includes data monitoring circuitry 210, rule maintenance circuitry 212, smart data transfer & storage circuitry 214, communications circuitry 208, input/output circuitry 206, processor 202, and/or a combination thereof, that terminates the continuous transfer in real-time of each subsequently-received health data. In some embodiments, the apparatus 200 continues to store each portion of received-health data. In some embodiments, the termination of continuous transfer in real-time cause storage of subsequently-received real-time health data without transferring such data to a long-term storage immediately, and/or only transferring such data at defined times and/o conditions as described with respect to FIGS. 8 and 9, for example. In this regard, termination of the real-time transfer reduces computing resource utilized for transmission and/or data storage via the external system.

### Conclusion

In some embodiments, some of the operations above may be modified or further amplified. Furthermore, in some embodiments, additional optional operations may be included. Modifications, amplifications, or additions to the operations above may be performed in any order and in any combination.

Many modifications and other embodiments of the disclosure set forth herein will come to mind to one skilled in the art to which this disclosure pertains having the benefit of the teachings presented in the foregoing description and the associated drawings. Therefore, it is to be understood that the embodiments are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

Although an example processing system has been described above, implementations of the subject matter and the functional operations described herein can be implemented in other types of digital electronic circuitry, or in computer software, firmware, or hardware, including the structures disclosed in this specification and their structural equivalents, or in combinations of one or more of them.

Embodiments of the subject matter and the operations described herein can be implemented in digital electronic circuitry, or in computer software, firmware, or hardware, including the structures disclosed in this specification and their structural equivalents, or in combinations of one or more of them. Embodiments of the subject matter described herein can be implemented as one or more computer programs, i.e., one or more modules of computer program instructions, encoded on computer storage medium for execution by, or to control the operation of, information/data processing apparatus. Alternatively, or in addition, the program instructions can be encoded on an artificially-generated propagated signal, e.g., a machine-generated electrical, optical, or electromagnetic signal, which is generated to encode information/data for transmission to suitable receiver apparatus for execution by an information/data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., multiple CDs, disks, or other storage devices).

The operations described herein can be implemented as operations performed by an information/data processing apparatus on information/data stored on one or more computer-readable storage devices or received from other sources.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus can also include, in addition to hardware, code that creates an execution environment for the computer program in question, e.g., code that constitutes processor firmware, a protocol stack, a repository management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or information/data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communication network.

The processes and logic flows described herein can be performed by one or more programmable processors executing one or more computer programs to perform actions by operating on input information/data and generating output. Processors suitable for the execution of a computer program include, by way of example, both general and special purpose microprocessors, and any one or more processors of any kind of digital computer. Generally, a processor will receive instructions and information/data from a read-only memory or a random access memory or both. The essential elements of a computer are a processor for performing actions in accordance with instructions and one or more memory devices for storing instructions and data. Generally, a computer will also include, or be operatively coupled to receive information/data from or transfer information/data to, or both, one or more mass storage devices for storing data, e.g., magnetic, magneto-optical disks, or optical disks. However, a computer need not have such devices. Devices suitable for storing computer program instructions and information/data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, embodiments of the subject matter described herein can be implemented on a computer having a display device, e.g., a CRT (cathode ray tube) or LCD (liquid crystal display) monitor, for displaying information/data to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described herein can be implemented in a computing system that includes a back-end component, e.g., as an information/data server, or that includes a middleware component, e.g., an application server, or that includes a front-end component, e.g., a client computer having a graphical user interface or a web browser through which a user can interact with an implementation of the subject matter described herein, or any combination of one or more such back-end, middleware, or front-end components. The components of the system can be interconnected by any form or medium of digital information/data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and typically interact through a communication network. The relationship of client and server arises by virtue of computer programs running on the respective computers and having a client-server relationship to each other. In some embodiments, a server transmits information/data (e.g., an HTML page) to a client device (e.g., for purposes of displaying information/data to and receiving user input from a user interacting with the client device). Information/data generated at the client device (e.g., a result of the user interaction) can be received from the client device at the server.

While this specification contains many specific implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

1. A computer-implemented method for smart health monitor operation comprising:
receiving real-time health data captured via a health monitor;
applying the real-time health data to at least one exception rule that determines an exception indicator; and
determining, based at least in part on the exception indicator, whether to transfer at least a portion of stored health data to an external system.

2. The computer-implemented method of claim 1 performed at the health monitor.

3. The computer-implemented method of any one of claims 1-2, wherein determining whether to transfer at least the portion of stored health data comprises:
determining that the exception indicator indications an exception;
identifying a complete stored health data set from a local datastore; and
transferring the complete stored health data set from the local datastore.

4. The computer-implemented method of any one of claims 1-3, wherein the portion of stored health data comprises the real-time health data.

5. The computer-implemented method of any one of claims 1-4, further comprising:
storing the real-time health data to a local datastore, wherein the apparatus is caused to continuously overwrite a defined historical timeseries of health data in the local datastore.

6. The computer-implemented method of any one of claims 1-5, wherein
determining whether to transfer at least the portion of stored health data comprises:
determining that the exception indicator indicates an exception;
in response to determining the exception indicator indicates the exception, transferring at least the portion of stored health data to the external system; and
in response to determining the exception indicator indicates the exception, continuously transferring, in real-time, of each subsequently-received real-time health data.

7. The computer-implemented method of claim 6, further comprising:
receiving a termination request; and
terminating the continuous transfer in real-time of each subsequently-received real-time health data.

8. The computer-implemented method of any one of claims 1-7, further comprising:
receiving annotated health data associated with the health monitor;
training a health rules generation model based at least in part on the annotated health data; and
generating the at least one exception rule utilizing the health rules generation model.

9. The computer-implemented method of any one of claims 1-8, further comprising:
generating the at least one exception rule utilizing a health rules generation model, the health rules generation model comprising a specially trained artificial intelligence or a specially configured machine-learning model.

10. The computer-implemented method of any one of claims 1-9, wherein the real-time health data comprises a plurality of data values associated with a plurality of different health parameters, the real-time health data comprising a particular timestamp.

11. The computer-implemented method of any one of claims 1-10, further comprising causing the external system to process at least the portion of stored health data to generate a health determination based at least in part on the portion of stored health data.

12. The computer-implemented method of any one of claims 1-11, wherein
determining whether to transfer at least the portion of stored health data comprising:
determining the exception indicator indicates no exception; and
storing the real-time health data to a local datastore without transfer to the external system.

13. The computer-implemented method of any one of claims 1-12, further
comprising:
tracking a timestamp interval associated with a time since last data transfer or a time since last default data transfer;
determining the timestamp interval satisfies a time interval threshold;
in response to determining the timestamp interval satisfies the time interval threshold:
identifying at least default health data; and
transferring at least the default transfer data to the external data system.

14. An apparatus comprising at least one processor and at least one memory including computer program code, the at least one memory and the computer program code configured to, with the at least one processor, cause the apparatus to perform any one of the methods according to claims 1-13.

15. A computer program product comprising at least one non-transitory computer-readable medium having computer-readable program instructions stored therein, the computer-readable program instructions comprising instructions, which when performed by an apparatus, are configured to cause the apparatus to at least perform any one of the methods according to claims 1-13.
